# FASCICULE DE BREVET EUROPEEN

(11) **EP 1 720 550 B1**
(45) Date de publication et mention de la délivrance du brevet: **15.04.2009**
(21) Numéro de dépôt: 05732905.4
(22) Date de dépôt: 25.02.2005
(51) Int. Cl.: A61K 31/4468, A61K 31/445, C07D 207/09, C07D 207/04, C07D 211/58

(54) **DERIVES DE PIPERIDINYLALKYLCARBAMATES, LEUR PREPARATION ET LEUR APPLICATION COMME INHIBITEURS DE L'ENZYME FAAH**
DERIVATE VON PIPERIDINYLALKYLCARBAMATEN, HERSTELLUNGSVERFAHREN DAVON UND VERWENDUNG DERSELBEN ALS FAAH-ENZYM-INHIBITOREN
DERIVATIVES OF PIPERIDINYLALKYLCARBAMATES, PREPARATION METHOD THEREOF AND USE OF SAME AS FAAH ENZYME INHIBITORS

(30) Priorité: 26.02.2004 FR 0401952
(43) Date de publication de la demande: 15.11.2006
(73) Titulaire: Sanofi-Aventis, 75013 Paris (FR)
(72) Inventeur: ABOUABDELLAH, Ahmed, F-94320 Thiais (FR); ALMARIO GARCIA, Antonio, F-92290 Chatenay Malabry (FR); HOORNAERT, Christian, F-92160 Antony (FR); JEUNESSE, Jean, F-75018 Paris (FR)
(74) Mandataire: Morel-Pécheux, Muriel
(86) Numéro de dépôt international: PCT/FR2005/000452
(87) Numéro de publication internationale: WO 2005/089759

(56) Documents cités:
- WO-A-99/26584
- WO-A-02/087569
- WO-A-03/065989
- WO-A-20/04020430
- WO-A-20/04067498
- WO-A-20/04099176
- WO-A-20/05033066
- US-A- 4 539 323
- SHAPIRO S L ET AL: "Aminoalkylamides and Oxazolidinediones" JOURNAL OF THE AMERICAN CHEMICAL SOCIETY, AMERICAN CHEMICAL SOCIETY, WASHINGTON, DC, US, vol. 81, no. 12, 1959, pages 3083-3088, XP002285705 ISSN: 0002-7863

## Description

L'invention a pour objet des dérivés de pipéridinylalkylcarbamates, leur préparation et leur application en thérapeutique.

On connaît déjà des dérivés de phénylalkylcarbamates, de dioxane-2-alkylcarbamates et de pipéridinyl- et pipérazinyl-alkylcarbamates, décrits respectivement dans les documents W02004/067498 A, WO2004/020430 A et WO2004/099176, inhibiteurs de l'enzyme FAAH (Fatty Acid Amido Hydrolase). Il existe toujours une nécessité de trouver et de développer des produits inhibiteurs de l'enzyme FAAH. Les composés de l'invention répondent à ce but.

Les composés de l'invention répondent à la formule générale (I) 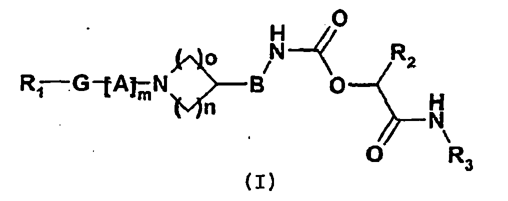 dans laquelle
m représente un nombre entier allant de 1 à 4 ;
n représente un nombre entier égal à 1, 2 ou 3 ;
o représente un nombre entier égal à 1 ou 2 ;
A est choisi parmi un ou plusieurs groupes X, Y et/ou Z ;
   X représente un groupe méthylène éventuellement substitué par un ou deux groupes C₁₋₆-alkyle, C₃₋₇-cycloalkyle ou C₃₋₇-cycloalkyl-C₁₋₃-alkylène;
   Y représente soit un groupe C₂-alcènylène éventuellement substitué par un ou deux groupes C₁₋₆ alkyle, C₃₋₇-cycloalkyle ou C₃₋₇-cycloalkyl-C₁₋₃-alkylène, soit un groupe C₂-alcynylène ;
   Z représente un groupe de formule :
   p représente un nombre entier allant de 1 à 5 ;
   q et r représentent des nombres entiers et sont définis tels que r+q soit un nombre allant de 1 à 5 ;
B représente une liaison covalente ou un groupe C₁₋₆₋alkylène ;
G représente une liaison covalente, un atome d'oxygène ou de soufre ou un groupe -CH(OH)-, CO, SO ou SO₂ ;
R₁ représente un groupe R₄ éventuellement substitué par un ou plusieurs groupes R₅ et/ou R₆ ;
   R₄ représente un groupe choisi parmi un furanyle, pyrrolyle, thiènyle, oxazolyle, isoxazolyle, thiazolyle, isothiazolyle, imidazolyle, pyrazolyle, thiadiazolyle, isothiadiazolyle, oxadiazolyle, thiadiazolyle, triazolyle, tétrazolyle, phényle, pyridinyle, pyridazinyle, pyrimidinyle, pyrazinyle, triazinyle, naphtalènyle, quinolinyle, tétrahydroquinolinyle, isoquinolinyle, tétrahydroisoquinolinyle, phtalazinyle, quinazolinyle, quinoxalinyle, cinnolinyle, naphthyridinyle, imidazopyrimidinyle, thiènopyrimidinyle, benzofuranyle, dihydrobenzofuranyle, benzothiènyle, dihydrobenzothiènyle, benzimidazolyle, benzoxazolyle, benzisoxazolyle, benzothiazolyle, benzisothiazolyle, indolyle, isoindolyle, indazolyle, pyrrolopyridinyle, furopyridinyle, dihydrofuropyridinyle, thiènopyridinyle, dihydrothiénopyridinyle, imidazopyridinyle, pyrazolopyridinyle, oxazolopyridinyle, isoxazolopyridinyle, thiazolopyridinyle ;
   R₅ représente un atome d'halogène, un groupe cyano, nitro, hydroxyle, C₁₋₆-alkyle, C₁₋₆-alcoxy, C₁₋₆-thioalkyle, C₁₋₆-fluoroalkyle, C₁₋₆-fluoroalcoxy, C₁₋₆-fluorothioalkyle, C₃₋₇-cycloalkyle, C₃₋₇-cycloalkyle-C₁₋₃-alkylène, NR₇R₈, NR₇COR₈, NR₇CO₂R₈, NR₇SO₂R₈, COR₇, CO₂R₇, CONR₇R₈, SO₂R₇, SO₂NR₇R₈ ou -O-(C₁₋₃-alkylène)-O- ;
   R₆ représente un groupe phényle, phényloxy, benzyloxy, pyridinyle, pyrazinyle, pyridazinyle, pyrimidinyle ou pyrimidinyloxy; le ou les groupes R₆ pouvant être substitués par un ou plusieurs groupes R₅ identiques ou différents l'un de l'autre ;
   R₇ et R₈ représentent indépendamment l'un de l'autre un atome d'hydrogène ou un groupe C₁₋₆-alkyle, ou forment avec le ou les atomes qui les portent un cycle choisi parmi un cycle azétidine, pyrrolidine, pipéridine, morpholine, thiomorpholine, azépine ou pipérazine, ce cycle étant éventuellement substitué par un groupe C₁₋₆-alkyle ou benzyle ;
R₂ représente un atome d'hydrogène ou un groupe C₁₋₆-alkyle ;
R₃ représente un atome d'hydrogène ou un groupe C₁₋₆-alkyle, C₂₋₇-cycloalkyle, C₃₋₇-cycloalkyle-C₁₋₃-alkylène.

Dans le cadre de l'invention, les composés de Formule générale (I) peuvent donc comporter plusieurs groupes A identiques ou différents entre eux.

Parmi les composés de formule générale (I), un premier sous-groupe de composés est constitué des composés pour lesquels :
m représente un nombre entier allant de 1 à 4 ;
n représente un nombre entier égal à 1 ou 2 ;
o représente un nombre entier égal à 1 ou 2;
A est choisi parmi un ou plusieurs groupes X et/ou Y ;
   X représente un groupe méthylène éventuellement substitué par un ou deux groupes C₁₋₆-alkyle, plus particulièrement
   méthyle;
   Y représente un groupe C₂-alcynylène ;
B représente une liaison covalente ou un groupe C₁₋₆-alkylène, plus particulièrement un méthylène ou un éthylène ;
G représente une liaison covalente ou un atome d'oxygène ;
R₁ représente un groupe R₄ éventuellement substitué par un ou plusieurs groupes R₅ et/ou R₆, plus particulièrement par un ou deux groupes R₅ et/ou R₆ ;
   R₄ représente un groupe choisi parmi un oxazolyle, isoxazolyle, thiazolyle, phényle, pyridinyle, naphtalènyle, quinolinyle, isoquinolinyle ;
   R₅ représente un atome d'halogène, plus particulièrement un chlore, un brome ou un fluor, un groupe cyano, NR₇R₈, un groupe C₁₋₆-alkyle, plus particulièrement un méthyle ou un isopropyle, C₁₋₆-alcoxy, plus particulièrement un méthoxy ou un éthoxy, C₁₋₆-fluoroalkyle, plus particulièrement un trifluorométhyle, ou C₁₋₆-fluoroalcoxy, plus particulièrement un trifluorométhoxy ;
   R₆ représente un groupe phényle, phényloxy ou pyrimidinyloxy; le ou les groupes R₆ pouvant être substitués par un ou plusieurs groupes R₅ identiques ou différents l'un de l'autre ;
   R₇ et R₈ représentent indépendamment l'un de l'autre un groupe C₁₋₆-alkyle, plus particulièrement un méthyle ;
R₂ représente.un atome d'hydrogène ou un groupe C₁₋₆-alkyle ;
R₃ représente un atome d'hydrogène ou un groupe C₁₋₆-alkyle, C₃₋₇-cycloalkyle, C₃₋₇-cycloalkyle-C₁₋₃-alkylène.

Parmi les composés de formule générale (I) et du premier sous-groupe, un second sous-groupe de composés est constitué des composés pour lesquels :
n, o, A, B, G, R₁, R₂ et R₃ sont tels que définis dans la formule (I) ou dans le sous-groupe ci-dessus ;
m représente un nombre entier égal à 1 ou 2, plus particulièrement égal à 1.

Parmi les composés de formule générale (I) et des sous-groupes ci-dessus, un troisième sous-groupe de composés est constitué des composés pour lesquels :
m, A, B, G, R₁, R₂ et R₃ sont tels que définis dans la formule (I) ou dans les sous-groupes ci-dessus ;
n est égal à 2 ;
o est égal à 2.

Parmi les composés de formule générale (I) et des sous-groupes ci-dessus, un quatrième sous-groupe de composés est constitué des composés pour lesquels :
m, n, o, A, B, G, R₂ et R₃ sont tels que définis dans la formule (I) ou dans les sous-groupes ci-dessus ;
R₁ représente un groupe R₄ éventuellement substitué par un ou plusieurs groupes R₅ et/ou R₆, plus particulièrement par un ou deux groupes R₅ et/ou R₆ ;
   R₄ représente un groupe choisi parmi un oxazolyle, isoxazolyle, phényle ou naphtalènyle ;
   R₅ représente un atome d'halogène, plus particulièrement un chlore, un brome ou un fluor, un groupe cyano, NR₇R₈, un groupe C₁₋₆-alkyle, plus particulièrement un méthyle ou un isopropyle, C₁₋₆-alcoxy, plus particulièrement un méthoxy ou un éthoxy, C₁₋₆-fluoroalkyle, plus particulièrement un trifluorométhyle, ou C₁₋₆-fluoroalcoxy, plus particulièrement un trifluorométhoxy ;
   R₆ représente un groupe phényle, phényloxy ou pyrimidinyloxy; le ou les groupes R₆ pouvant être substitués par un ou plusieurs groupes R₅ identiques ou différents l'un de l'autre ;
   R₇ et R₈ représentent indépendamment l'un de l'autre un groupe C₁₋₆-alkyle, plus particulièrement un méthyle.

Parmi les composés de formule générale (I), un cinquième sous-groupe de composés est constitué des composés pour lesquels :
m, n, o, A, B, G et R₁ sont tels que définis dans la formule (I) ou dans les sous-groupes ci-dessus ;
R₂ représente un atome d'hydrogène ;
R₃ représente un atome d'hydrogène ou un groupe C₁₋₆-alkyle, plus particulièrement un méthyle.

Parmi les composés de formule générale (I), les composés suivants peuvent être cités :
- {1-[(3,4'-difluorobiphényl-4-yl)méthyl]pipéridin-4-yl}méthylcarbamate de 2-(méthylamino)-2-oxoéthyle ;
- {1-[(3-chloro-4'-fluorobiphényl-4-yl)méthyl]pipéridin-4-yl}méthylcarbamate de 2-(méthylamino)-2-oxoéthyle ;
- {1-[3-(4-fluorophénoxy)benzyl]pipéridin-4-yl}méthylcarbamate de 2-(méthylamino)-2-oxoéthyle ;
- {1-[4-(4-chloro-3-fluorophénoxy)benzyl]pipéridin-4-yl}méthylcarbamate de 2-(méthylamino)-2-oxoéthyle ;
- 2-{1-[3-(trifluorométhoxy)benzyl]pipéridin-4-yl}éthylcarbamate de 2-(méthylamino)-2-oxoéthyle ;
- 2-{1-[4-(trifluorométhoxy)benzyl]pipéridin-4-yl}éthylcarbamate de 2-(méthylamino)-2-oxoéthyle ;
- 2-[1-(3-*tert*-butoxybenzyl)pipéridin-4-yl]éthylcarbamate de 2-(méthylamino)-2-oxoéthyle ;
- 2-[1-(3-*tert*-butoxybenzyl)pipéridin-4-yl]méthylcarbamate de 2-(méthylamino)-2-oxoéthyle ;
- 2-[1-(2,4-dichlorobenzyl)pipéridin-4-yl]éthylcarbamate de 2-(méthylamino)-2-oxoéthyle ;
- 2-[1-(2,5-dichlorobenzyl)pipéridin-4-yl]éthylcarbamate de 2-(méthylamino)-2-oxoéthyle ;
- 2-[1-(3,5-dichlorobenzyl)pipéridin-4-yl]éthylcarbamate de 2-(méthylamino)-2-oxoéthyle ;
- 2-[1-(2-chloro-5-fluorobenzyl)pipéridin-4-yl]éthylcarbamate de 2-(méthylamino)-2-oxoéthyle ;
- 2-[1-(3-chloro-2-fluorobenzyl)pipéridin-4-yl]éthylcarbamate de 2-(méthylamino)-2-oxoéthyle ;
- 2-[1-(3-chloro-5-méthylbenzyl)pipéridin-4-yl]éthylcarbamate de 2-(méthylamino)-2-oxoéthyle ;
- 2-{1-[(3,4'-difluorobiphényl-4-yl)méthyl]pipéridin-4-yl}éthylcarbamate de 2-(méthylamino)-2-oxoéthyle ;
- 2-{1-[(3-chloro-4'-fluorobiphényl-4-yl)méthyl]pipéridin-4-yl}éthylcarbamate de 2-(méthylamino)-2-oxoéthyle ;
- 2-{1-[4-(4-chloro-3-fluorophénoxy)benzyl]pipéridin-4-yl}éthylcarbamate de 2-(méthylamino)-2-oxoéthyle ;
- {1-[3-(4-fluorophénoxy)benzyl]pipéridin-4-yl}carbamate de 2-(méthylamino)-2-oxoéthyle ;
- {1-[3-(trifluorométhoxy)benzyl]pipéridin-4-yl}méthylcarbamate de 2-(méthylamino)-2-oxoéthyle ;
- {1-[4-(trifluorométhoxy)benzyl]pipéridin-4-yl}méthylcarbamate de 2-(méthylamino)-2-oxoéthyle ;
- {1-[3-(pyrimidin-2-yloxy)benzyl]pipéridin-4-yl}méthylcarbamate de 2-(méthylamino)-2-oxoéthyle ;
- 2-[1-(2-chloro-4-fluorobenzyl)pipéridin-4-yl]éthylcarbamate de 2-(méthylamino)-2-oxoéthyle ;
- 2-[1-(3-chloro-4-fluorobenzyl)pipéridin-4-yl]éthylcarbamate de 2-(méthylamino)-2-oxoéthyle ;
- 2-[1-(3-cyano-5-fluorobenzyl)pipéridin-4-yl]éthylcarbamate de 2-(méthylamino)-2-oxoéthyle ;
- 2-{1-[3-(4-fluorophénoxy)benzyl]pipéridin-4-yl}éthylcarbamate de 2-(méthylamino)-2-oxoéthyle ;
- (1-{[3-(4-chlorophényl)isoxazol-5-yl]méthyl}pipéridin-4-yl)méthylcarbamate de 2-(méthylamino)-2-oxoéthyle ;
- (1-{[5-(4-chlorophényl)-1,3-oxazol-2-yl]méthyl}pipéridin-4-yl)méthylcarbamate de 2-(méthylamino)-2-oxoéthyle ;
- [1-({4-[4-(trifluorométhyl)phényl]-1,3-thiazol-2-yl}méthyl)pipéridin-4-yl]méthylcarbamate de 2-(méthylamino)-2-oxoéthyle ;
- 2-(1-{[3-(4-chlorophényl)isoxazol-5-yl]méthyl}pipéridin-4-yl)éthylcarbamate de 2-(méthylamino)-2-oxoéthyle ;
- 2-(1-{[5-(4-chlorophényl)-1,3-oxazol-2-yl]méthyl}pipéridin-4-yl)éthylcarbamate de 2-(méthylamino)-2-oxoéthyle ;
- 2-[1-({4-[4-(trifluorométhyl)phényl]-1,3-thiazol-2-yl}méthyl)pipéridin-4-yl]éthylcarbamate de 2-(méthylamino)-2-oxoéthyle ;
- (1-{2-[5-(4-chlorophényl)isoxazol-3-yl]éthyl}pipéridin-4-yl)méthylcarbamate de 2-(méthylamino)-2-oxoéthyle ;
- 2-(1-{2-[3-(4-chlorophényl)isoxazol-5-yl]éthyl}pipéridin-4-yl)éthylcarbamate de 2-(méthylamino)-2-oxoéthyle ;
- (1-{2-[3-(4-chlorophényl)isoxazol-5-yl]éthyl}pipéridin-4-yl)méthylcarbamate de 2-(méthylamino)-2-oxoéthyle ;
- (1-{3-[3-(4-chlorophényl)isoxazol-5-yl]propyl}pipéridin-4-yl)méthylcarbamate de 2-(méthylamino)-2-oxoéthyle ;
- (1-{3-[5-(4-chlorophényl)isoxazol-3-yl]propyl}pipéridin-4-yl)méthylcarbamate de 2-(méthylamino)-2-oxoéthyle ;
- {1-[1-(2-chloro-4-fluorophényl)éthyl]pipéridin-4-yl}méthylcarbamate de 2-(méthylamino)-2-oxoéthyle ;
- (1-{1-[3-(4-chlorophénoxy)phényl]éthyl}pipéridin-4-yl)méthylcarbamate de 2-(méthylamino)-2-oxoéthyle ;
- (1-{1-[2-chloro-3-(4-chlorophénoxy)phényl]éthyl}pipéridin-4-yl)méthylcarbamate de 2-(méthylamino)-2-oxoéthyle ;
- 2-(1-{1-[3-(trifluorométhoxy)phényl]éthyl}pipéridin-4-yl)éthylcarbamate de 2-(méthylamino)-2-oxoéthyle ;
- 2-{1-[1-(2-chloro-4-fluorophényl)éthyl]pipéridin-4-yl}éthylcarbamate de 2-(méthylamino)-2-oxoéthyle ;
- {1-[4-(4-chlorophényl)but-3-yn-1-yl]pipéridin-4-yl}carbamate de 2-(méthylamino)-2-oxoéthyle ;
- (1-[5-(4-chlorophényl)pent-4-yn-1-yl]pipéridin-4-yl)carbamate de 2-(méthylamino)-2-oxoéthyle ;
- {1-[5-(2,5-dichlorophényl)pent-4-yn-1-yl]pipéridin-4-yl}carbamate de 2-(méthylamino)-2-oxoéthyle ;
- {1-[4-(4-chlorophényl)but-3-yn-1-yl]pipéridin-4-yl}méthylcarbamate de 2-(méthylamino)-2-oxoéthyle ;
- {1-[4-(4-chloro-2-fluorophényl)but-3-yn-1-yl]pipéridin-4-yl}méthylcarbamate de 2-(méthylamino)-2-oxoéthyle ;
- {1-[4-(2,5-dichlorophényl)but-3-yn-1-yl]pipéridin-4-yl}méthylcarbamate de 2-(méthylamino)-2-oxoéthyle.

Parmi les composés de formule générale (I), une sous-famille de composés est constituée des composés répondant à la formule générale (I') : 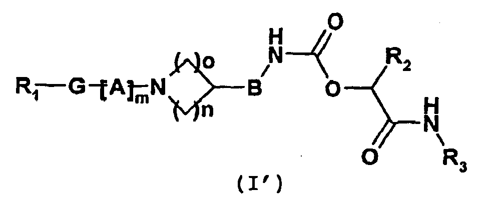 dans laquelle
m représente un nombre entier allant de 1 à 4 ;
n représente un nombre entier égal à 1, 2 ou 3 ;
o représente un nombre entier égal à 1 ou 2;
A est choisi parmi un ou plusieurs groupes X, Y et/ou Z ;
   X représente un groupe méthylène éventuellement substitué par un ou deux groupes C₁₋₆-alkyle, C₃₋₇-cycloalkyle ou C₃₋₇-cycloalkyl-C₁₋₃-alkylène;
   Y représente soit un groupe C₂-alcènylène éventuellement substitué par un ou deux groupes C₁₋₆ alkyle, C₃₋₇-cycloalkyle ou C₃₋₇-cycloalkyl-C₁₋₃-alkylène; soit un groupe C₂-alcynylène ;
   Z représente un groupe de formule : 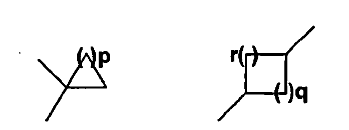
   p représente un nombre entier allant de 1 à 5 ;
   q et r représentent des nombres entiers et sont définis tels que r+q soit un nombre allant de 1 à 5 ;
B représente une liaison covalente ou un groupe C₁₋₆-alkylène ;
G représente une liaison covalente, un atome d'oxygène ou de soufre ou un groupe -CH(OH)-, CO, SO ou SO₂ ;
R₁ représente un groupe R₄ éventuellement substitué par un ou plusieurs groupes R₅ et/ou R₆ ;
   R₄ représente un groupe choisi parmi un furanyle, pyrrolyle, thiènyle, oxazolyle, isoxazolyle, thiazolyle, isothiazolyle, imidazolyle, pyrazolyle, thiadiazolyle, isothiadiazolyle, oxadiazolyle, thiadiazolyle, triazolyle, tétrazolyle, phényle, pyridinyle, pyridazinyle, pyrimidinyle, pyrazinyle, triazinyle, naphtalènyle, quinolinyle, tétrahydroquinolinyle, isoquinolinyle, tétrahydroisoquinolinyle, phtalazinyle, quinazolinyle, quinoxalinyle, cinnolinyle, naphthyridinyle, imidazopyrimidinyle, thiènopyrimidinyle, benzofuranyle, dihydrobenzofuranyle, benzothiènyle, dihydrobenzothiènyle, benzimidazolyle, benzoxazolyle, benzisoxazolyle, benzothiazolyle, benzisothiazolyle, indolyle, isoindolyle, indazolyle, pyrrolopyridinyle, furopyridinyle, dihydrofuropyridinyle, thiènopyridinyle, dihydrothiénopyridinyle, imidazopyridinyle, pyrazolopyridinyle, oxazolopyridinyle, isoxazolopyridinyle, thiazolopyridinyle ;
   R₅ représente un atome d'halogène, un groupe cyano, nitro, hydroxyle, C₁₋₆-alkyle, C₁₋₆-alcoxy, C₁₋₆-thioalkyle, C₁₋₆-fluoroalkyle, C₁₋₆-fluoroalcoxy, C₁₋₆-fluorothioalkyle, C₃₋₇-cycloalkyle, C₃₋₇-cycloalkyle-C₁₋₃-alkylène, NR₇R₈, NR₇COR₈, NR₇CO₂R₈, NR₇SO₂R₈, COR₇, CO₂R₇, CONR₇R₈, SO₂R₇, SO₂NR₇R₈ ou -O-(C₁₋₃-alkylène)-O- ;
   R₆ représente un groupe phényle, phényloxy, benzyloxy, pyridinyle, pyrazinyle, pyridazinyle ou pyrimidinyle; le ou les groupes R₆ pouvant être substitués par un ou plusieurs groupes R₅ identiques ou différents l'un de l'autre ;
   R₇ et R₈ représentent indépendamment l'un de l'autre un atome d'hydrogène ou un groupe C₁₋₆-alkyle, ou forment avec le ou les atomes qui les portent un cycle choisi parmi un cycle azétidine, pyrrolidine, pipéridine, morpholine, thiomorpholine, azépine ou pipérazine, ce cycle étant éventuellement substitué par un groupe C₁₋₆-alkyle ou benzyle ;
R₂ représente un atome d'hydrogène ou un groupe C₁₋₆-alkyle ;
R₃ représente un atome d'hydrogène ou un groupe C₁₋₆-alkyle, C₃₋₇-cycloalkyle, C₃₋₇-cycloalkyle-C₁₋₃-alkylène.

Parmi les composés de formule générale (I'), un premier sous-groupe de composés est constitué des composés pour lesquels :
m représente un nombre entier allant de 1 à 3 ;
n représente un nombre entier égal à 1 ou 2 ;
o représente un nombre entier égal à 2;
A est un groupe méthylène ;
B représente une liaison covalente ou un groupe C₁₋₆-alkylène, plus particulièrement un méthylène ou un éthylène ;
G représente une liaison covalente ou un atome d'oxygène ;
R₁ représente un groupe R₄ éventuellement substitué par un ou plusieurs groupes R₅ et/ou R₆, plus particulièrement par un ou deux groupes R₅ et/ou R₆ ;
   R₄ représente un groupe choisi parmi un phényle, pyridinyle, naphtalènyle, isoquinolinyle ;
   R₅ représente un atome d'halogène, plus particulièrement un chlore, un brome ou un fluor, un groupe cyano, un groupe N,N-diméthylamino, un groupe C₁₋₆-alkyle, plus particulièrement un isopropyle, C₁₋₆-alcoxy, plus particulièrement un méthoxy ou un éthoxy, C₁₋₆-fluoroalkyle, plus particulièrement un trifluorométhyle ;
   R₆ représente un groupe phényle;
R₂ représente un atome d'hydrogène ou un groupe C₁₋₆-alkyle ;
R₃ représente un atome d'hydrogène ou un groupe C₁₋₆-alkyle, C₃₋₇-cycloalkyle, C₃₋₇-cycloalkyle-C₁₋₃-alkylène.

Parmi les composés de formule générale (I'), un second sous-groupe de composés est constitué des composés pour lesquels :
m, n, o, A, B, G et R₁ sont tels que définis dans le sous-groupe 1 ;
R₂ représente un atome d'hydrogène ;
R₃ représente un atome d'hydrogène ou un groupe C₁₋₆-alkyle, plus particulièrement un méthyle.

Parmi les composés de formule générale (I'), les composés suivants peuvent être cités :
- {1-[(2-chlorophényl)méthyl]pipéridin-4-yl}méthylcarbamate de 2-(méthylamino)-2-oxoéthyle ;
- {1-[(4-chlorophényl)méthyl]pipéridin-4-yl}méthylcarbamate de 2-(méthylamino)-2-oxoéthyle ;
- {1-[(4-chlorophényl)méthyl]pipéridin-4-yl}carbamate de 2-(méthylamino)-2-oxoéthyle ;
- (1-[4-(1-méthyléthyl)phényl]méthyl pipéridin-4-yl)méthylcarbamate de 2-(méthylamino)-2-oxoéthyle;
- [1-(biphényl-4-ylméthyl)pipéridin-4-yl]carbamate de 2-(méthyl amino)-2-oxoéthyle ;
- [1-(biphényl-4-ylméthyl)pipéridin-4-yl]méthylcarbamate de 2-(méthylamino)-2-oxoéthyle ;
- 2-[1-(biphényl-4-ylméthyl)pipéridin-4-yl]éthylcarbamate de 2- (méthylamino) -2-oxoéthyle ;
- [1-(2-biphényl-4-yléthyl)pipéridin-4-yl]carbamate de 2-(méthyl amino)-2-oxoéthyle ;
- [1-(naphthalèn-2-ylméthyl)pipéridin-4-yl]méthylcarbamate de 2-(méthylamino)-2-oxoéthyle ;
- 2-{1-[(4-bromophényl)méthyl]pipéridin-4-yl}éthylcarbamate de 2-(méthylamino)-2-oxoéthyle ;
- 2- (1-{[3-(trifluorométhyl)phényl]méthyl}pipéridin-4-yl)éthylcarbamate de 2-(méthylamino)-2-oxoéthyle ;
- 2-(1-{[4-(trifluorométhyl)phényl]méthyl}pipéridin-4-yl)éthylcarbamate de 2-(méthylamino)-2-oxoéthyle ;
- 2-{1-[(2,3-dichlorophényl)méthyl]pipéridin-4-yl}éthylcarbamate de 2-(méthylamino)-2-oxoéthyle ;
- 2-{1-[(3,4-dichlorophényl)méthyl]pipéridin-4-yl}éthylcarbamate de 2-(méthylamino)-2-oxoéthyle ;
- 2-[1-(naphthalèn-1-ylméthyl)pipéridin-4-yl]éthylcarbamate de 2-(méthylamino)-2-oxoéthyle ;
- 2-[1-(naphthalèn-2-ylméthyl)pipéridin-4-yl]éthylcarbamate de 2-(méthylamino)-2-oxoéthyle ;
- 2-[1-(pyridin-2-ylméthyl)pipéridin-4-yl]éthylcarbamate de 2-(méthylamino)-2-oxoéthyle;
- (1-{2-[4-fluoro-2-(méthyloxy)phényl]éthyl}pipéridin-4-yl)méthylcarbamate de 2-(méthylamino)-2-oxoéthyle ;
- (1-{2-[(4-fluorophényl)oxy]éthyl}pipéridin-4-yl)carbamate de 2-(méthylamino)-2-oxoéthyle ;
- (1-{2-[(4-chlorophényl)oxy]éthyl}pipéridin-4-yl)carbamate de 2-(méthylamino)-2-oxoéthyle ;
- (1-{2-[(2,4-dichlorophényl)oxy]éthyl}pipéridin-4-yl)carbamate de 2-(méthylamino)-2-oxoéthyle ;
- (1-{2-[(4-chlorophényl)oxy]éthyl}pipéridin-4-yl)méthylcarbamate de 2-(méthylamino)-2-oxoéthyle ;
- (1-{2-[(2,4-dichlorophényl)oxy]éthyl} pipéridin-4-yl)méthylcarbamate de 2-(méthylamino)-2-oxoéthyle ;
- 2-(1-{2-[(4-fluorophényl)oxy]éthyl}pipéridin-4-yl)éthylcarbamate de 2-(méthylamino)-2-oxoéthyle ;
- 2-(1-{2-[(4-chlorophényl)oxy]éthyl}pipéridin-4-yl)éthylcarbamate de 2-(méthylamino)-2-oxoéthyle ;
- (1-{2-[(4-fluorophényl)oxy]éthyl} pyrrolidin-3-yl)méthylcarbamate de 2-(méthylamino)-2-oxoéthyle ;
- (1-{2-[(4-chlorophényl)oxy]éthyl}pyrrolidin-3-yl)méthylcarbamate de 2-(méthylamino)-2-oxoéthyle ;
- (1-{2-[3-(trifluorométhyl)phényl]éthyl}pipéridin-4-yl) carbamate de 2-(méthylamino)-2-oxoéthyle ;
- {1-[2-(4-chlorophényl)éthyl]pipéridin-4-yl}carbamate de 2-(méthylamino)-2-oxoéthyle ;
- {1-[2-(4-cyanophényl)éthyl]pipéridin-4-yl}carbamate de 2-(méthylamino)-2-oxoéthyle ;
- (1-{2-[(isoquinolin-5-yl)oxy]éthyl}pipéridin-4-yl)carbamate de 2-(méthylamino)-2-oxoéthyle ;
- [1-(2-naphthalèn-1-yléthyl)pipéridin-4-yl]carbamate de 2-(méthylamino)-2-oxoéthyle ;
- [1-(2-naphthalèn-2-yléthyl)pipéridin-4-yl]carbamate de 2-(méthylamino)-2-oxoéthyle ;
- {1-[3-(4-chlorophényl)propyl]pipéridin-4-yl}carbamate de 2-(méthylamino)-2-oxoéthyle ;
- (1-{3-[4-(méthyloxy)phényl]propyl}pipéridin-4-yl)carbamate de 2-(méthylamino)-2-oxoéthyle ;
- {1-[2-(3-chlorophényl)éthyl]pipéridin-4-yl}méthylcarbamate de 2-(méthylamino)-2-oxoéthyle ;
- (1-{2-[4-(éthyloxy)phényl]éthyl}pipéridin-4-yl)méthylcarbamate de 2-(méthylamino)-2-oxoéthyle ;
- (1-{2-[4-(diméthylamino)phényl]éthyl}pipéridin-4-yl)méthylcarbamate de 2-(méthylamino)-2-oxoéthyle ;
- {1-[2-(2,4-dichlorophényl)éthyl]pipéridin-4-yl}[méthylcarbamate] de 2-(méthylamino)-2-oxoéthyle ;
- [1-(2-naphthalèn-1-yléthyl)pipéridin-4-yl]méthylcarbamate de 2-(méthylamino)-2-oxoéthyle ;
- [1-(2-naphthalèn-2-yléthyl)pipéridin-4-yl]méthylcarbamate de 2-(méthylamino)-2-oxoéthyle ;
- (1-{3-[4-(méthyloxy)phényl]propyl}pipéridin-4-yl)méthylcarbamate de 2-(méthylamino)-2-oxoéthyle ;
- 2{1-[2-(2-chlorophényl)éthyl]pipéridin-4-yl}éthylcarbamate de 2-(méthylamino)-2-oxoéthyle ;
- 2-{1-[2-(4-fluorophényl)éthyl]pipéridin-4-yl}éthylcarbamate de 2-(méthylamino)-2-oxoéthyle ;
- 2-(1-{2-[4-(éthyloxy)phényl]éthyl}pipéridin-4-yl)éthylcarbamate de 2-(méthylamino)-2-oxoéthyle ;
- 2-{1-[2-(2-chloro-6-fluorophényl)éthyl]pipéridin-4-yl}éthylcarbamate de 2-(méthylamino)-2-oxoéthyle ;
- 2-(1-{3-[4-(méthyloxy)phényl]propyl}pipéridin-4-yl)éthylcarbamate de 2-(méthylamino)-2-oxoéthyle.

Les composés de formule générale (I) peuvent comporter un ou plusieurs carbones asymétriques. Ils peuvent exister sous forme d'énantiomères ou de diastéréoisomères. Ces énantiomères et diastéréoisomères, ainsi que leurs mélanges, y compris les mélanges racémiques, font partie de l'invention.

Les composés de formule (I) peuvent exister à l'état de bases ou de sels d'addition à des acides. De tels sels d'addition font partie de l'invention.

Ces sels sont avantageusement préparés avec des acides pharmaceutiquement acceptables, mais les sels d'autres acides utiles, par exemple, pour la purification ou l'isolement des composés de formule (I) font également partie de l'invention.
Les composés de formule générale (I) peuvent se trouver sous forme d'hydrates ou de solvats, à savoir sous forme d'associations ou de combinaisons avec une ou plusieurs molécules d'eau ou avec un solvant. De tels hydrates et solvats font également partie de l'invention.

Dans le cadre de l'invention, on entend par :
- C_{t-z} où t et z peuvent prendre les valeurs de 1 à 7, une chaîne carbonée pouvant avoir de t à z atomes de carbone, par exemple C₁₋₃ une chaîne carbonée qui peut avoir de 1 à 3 atomes de carbone;
- alkyle, un groupe aliphatique saturé, linéaire ou ramifié; par exemple un groupe C₁₋₆-alkyle représente une chaîne carbonée de 1 à 6 atomes de carbone, linéaire ou ramifiée, plus particulièrement un méthyle, éthyle, propyle, isopropyle, butyle, isobutyle, tertbutyle, pentyle, hexyle ;
- alkylène, un groupe alkyle divalent saturé, linéaire ou ramifié, par exemple un groupe C₁₋₃-alkylène représente une chaîne carbonée divalente de 1 à 3 atomes de carbone, linéaire ou ramifiée, plus particulièrement un méthylène, éthylène, 1-méthyléthylène, propylène ;
- cycloalkyle, un groupe alkyle cyclique, par exemple un groupe C₃₋₇-cycloalkyle représente un groupe carboné cyclique de 3 à 7 atomes de carbone, plus particulièrement un cyclopropyle, cyclobutyle, cyclopentyle, cyclohexyle, cycloheptyle ;
- alcènylène, un groupe aliphatique à 2 carbones, insaturé divalent, plus particulièrement un éthylène ;
- C₂-alcynylène, un groupe -C≡C- ;
- alcoxy, un groupe -O-alkyle à chaîne aliphatique saturée, linéaire ou ramifiée ;
- thioalkyle, un groupe -S-alkyle à chaîne aliphatique saturée, linéaire ou ramifiée ;
- fluoroalkyle, un groupe alkyle dont un ou plusieurs atomes d'hydrogène ont été substitués par un atome de fluor ;
- fluoroalcoxy, un groupe alcoxy dont un ou plusieurs atomes d'hydrogène ont été substitués par un atome de fluor ;
- fluorothioalkyle, un groupe thioalkyle dont un ou plusieurs atomes d'hydrogène ont été substitués par un atome de fluor
- atome d'halogène, un fluor, un chlore, un brome ou un iode.

Dans ce qui suit, on entend par groupe protecteur Pg un groupe qui permet, d'une part, de protéger une fonction réactive telle qu'un hydroxy ou une amine pendant une synthèse et, d'autre part, de régénérer la fonction réactive intacte en fin de synthèse. Des exemples de groupes protecteurs ainsi que des méthodes de protection et de déprotection sont données dans « Protective Groups in Organic Synthesis », Green et al., 2nd Edition (John Wiley & Sons, Inc., New York).

Les composés de l'invention peuvent être préparés selon différentes méthodes, illustrées par le schéma 1 qui suit.

Ainsi une première méthode (schéma 1) consiste à faire réagir un composé de formule générale (II), dans laquelle B, R₂, n et o sont tels que définis dans la formule générale (I), avec un dérivé de formule générale (III) dans laquelle W représente un groupe mésylate, tosylate, ou un atome de chlore, de brome ou d'iode, et m, G, A et R₁ sont tels que définis dans la formule générale (I), en présence d'une base telle que la triéthylamine, l'hydrure de sodium, le tert-butoxyde de sodium ou le carbonate de sodium dans un solvant tel que le tétrahydrofurane, l'acétonitrile, le diméthylsulfoxyde ou le diméthylformamide à une température comprise entre 0°C et la température de reflux du solvant.

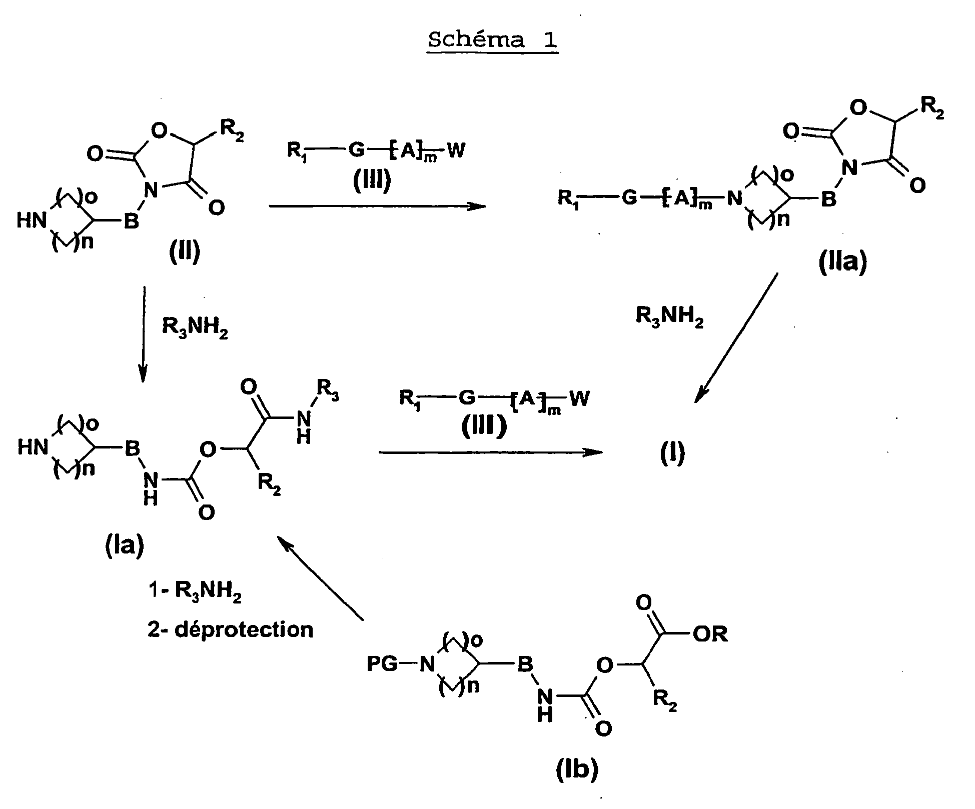

L'oxazolidine-dione de formule générale (IIa) ainsi obtenue est ensuite transformée en composé de formule générale (I), par aminolyse au moyen d'une amine de formule générale R₃NH₂ dans laquelle R₃ est tel que défini dans la formule générale (I). La réaction d'aminolyse peut être réalisée dans un solvant tel que le méthanol, l'éthanol ou un mélange de solvants tels que le méthanol et le tétrahydrofurane ou le méthanol et le dioxane.

Une variante d'obtention des composés de formule générale (I) (schéma 1) consiste à transformer un composé de formule générale (II) telle que définie ci-dessus par aminolyse, dans les conditions décrites ci-dessus, au moyen d'une amine de formule générale R₃NH₂ telle que définie ci-dessus pour obtenir un dérivé carbamate-amide de formule générale (Ia), dans laquelle B, R₂, R₃, n et o sont tels que définis dans la formule générale (I). Le composé de formule générale (I) est ensuite obtenu par réaction du composé (Ia) avec un dérivé de formule générale (III) tel que défini ci-dessus, dans les conditions décrites ci-dessus.
Le dérivé carbamate-amide de formule générale (Ia) telle que définie ci-dessus peut également être obtenu à partir du carbamate-ester de formule générale (Ib), dans laquelle B, R₂, n et o sont tels que définis dans la formule générale (I), PG représente un groupe protecteur tel qu'un Boc (*tert-*butyloxycarbonyl) et R représente un groupe méthyle ou éthyle, par aminolyse au moyen d'une amine de formule générale R₃NH₂ telle que définie ci-dessus et dans les conditions décrites ci-dessus, puis par déprotection, par exemple en présence d'une solution d'acide chlorhydrique (5N) dans l'isopropanol.

Les carbamates-esters (Ib) peuvent être préparés selon la méthode, illustrée par le schéma 2 qui suit.

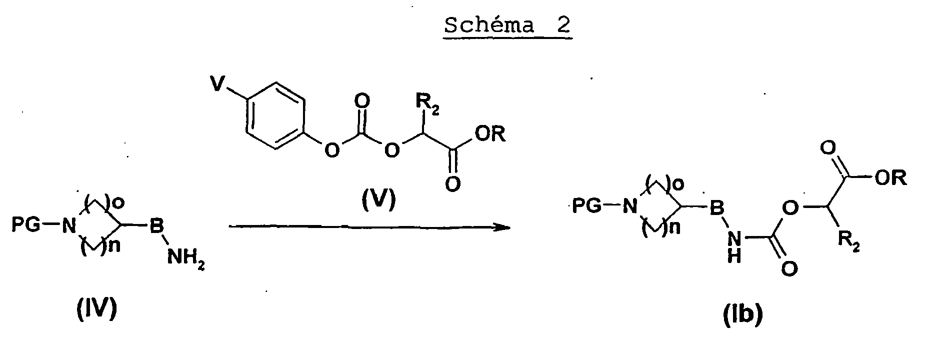

Selon le schéma 2, le carbamate-ester de formule générale (Ib) est obtenu en faisant réagir une amine de formule générale (IV), dans laquelle B, n et o sont tels que définis dans la formule générale (I) et PG représente un groupe protecteur tel qu'un Boc, avec un carbonate de formule générale (V) dans laquelle V représente un atome d'hydrogène ou un groupe nitro, R₂ est tel que défini dans la formule générale (I) et R représente un groupe méthyle ou éthyle.

Quand leur mode de préparation n'est pas décrit, les composés de formule générale (II) peuvent être préparés selon des méthodes qui sont décrites dans la littérature ou selon des méthodes analogues à celles décrites ou qui sont connues de l'homme du métier.
Les carbonates de formule générale (V) peuvent être préparés selon toute méthode décrite dans la littérature, par exemple par réaction d'un alcool de formule générale HOCHR₂COOR où R représente un groupe méthyle ou éthyle, avec le chloroformiate de phényle ou de 4-nitrophényle, en présence d'une base telle que la triéthylamine ou la diisopropyléthylamine.
Les composés de formules générales (III), (IV) et les amines de formule générale R₃NH₂ sont disponibles dans le commerce ou préparés selon des méthodes qui sont décrites dans la littérature ou qui sont connues de l'homme du métier.

Les composés de formule générale (Ia), dans laquelle B, R₂, R₃, n et o sont tels que définis dans la formule générale (I), sont nouveaux et font également partie de l'invention. Ils sont utiles en tant qu'intermédiaires de synthèse pour la préparation des composés de formule générale (I).

Les composés de formule générale (IIa), dans laquelle m, G, A, R₁, B, R₂, n et o sont tels que définis dans la formule générale (I), le composé 3-[1-(phénylméthyl)-4-pipéridinyl]-2,4-oxazolidinedione étant exclu, sont nouveaux et font également partie de l'invention. Ils sont utiles en tant qu'intermédiaires de synthèse pour la préparation des composés de formule générale (I).

Les exemples qui vont suivre illustrent la préparation de quelques composés de l'invention. Les microanalyses, les spectres I.R. et R.M.N. et/ou la LC-MS (Liquid Chromatography coupled to Mass Spectroscopy) confirment les structures et les puretés des composés obtenus.
PF(°C) représente le point de fusion en degrés Celsius.
Les numéros indiqués entre parenthèses dans les titres des exemples correspondent à ceux de la 1ère colonne du tableau ci-après.

### Exemple 1 (composé N°25)

### 2-[1-(biphényl-4-ylméthyl)pipéridin-4-yl]éthylcarbamate de 2-(méthylamino)-2-oxoéthyle

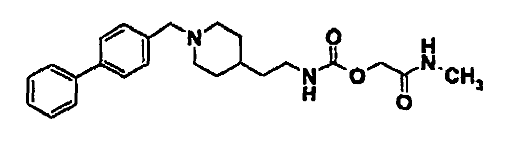

### 1.1. chlorhydrate de 3-(2-pipéridin-4-yléthyl)-1,3-oxazolidine-2,4-dione

A une solution de 10 g (77,40 mmoles) de 2-pipéridin-4-yléthanol, de 22,33 g (85,14 mmoles) de triphénylphosphine et de 9,39 g (92,88 mmoles) de 1,3-oxazolidin-2,4-dione (J. Med. Chem. 1991, 34, 1538-1544) dans 150 ml de tétrahydrofurane, refroidie à environ -10°C, on ajoute goutte à goutte, sous atmosphère inerte, une solution de 15,65 g (77,40 mmoles) de diisopropylazodicarboxylate (DIAD) dans 25 ml de tétrahydrofurane tout en maintenant la température du milieu réactionnel entre -10°C et 0°C. On poursuit l'agitation à 0°C pendant 1 heure, puis à 25°C pendant 22 heures. On collecte le solide formé par filtration, on le lave plusieurs fois par du tétrahydrofurane, puis on le sèche sous vide à environ 70°C On reprend ensuite ce solide avec une solution d'acide chlorhydrique (5N) dans l'isopropanol, On collecte le solide formé par filtration puis on le lave avec de l'acétate d'éthyle et de l'éther.
On obtient, après séchage sous vide à environ 70°C, 6,45 g de chlorhydrate sous forme de solide blanc.
PF (°C) : 178°C

### 1.2. 3-{2-[1-(biphényl-4-ylméthyl)pipéridin-4-yl]éthyl}-1,3-oxazolidine-2,4-dione

On porte au reflux pendant 17 heures une solution de 0,40 g (1,61 mmole) de chlorhydrate de 3-(2-pipéridin-4-yléthyl)-1,3-oxazolidine-2,4-dione, préparé à l'étape 1.1., de 0,326 g (1,61 mmole) de 4-(chlorométhyl)biphényle et de 0,51 g (4,82 mmoles) de carbonate de sodium, dans 3 ml d'acétonitrile. On laisse revenir à température ambiante, on filtre et on concentre le filtrat sous pression réduite. On reprend le résidu par du dichlorométhane et de l'eau, on sépare la phase aqueuse et on l'extrait deux fois avec du dichlorométhane. On lave les phases organiques réunies avec une solution aqueuse saturée en chlorure de sodium et on les sèche sur sulfate de sodium. Après évaporation du solvant, le résidu obtenu est purifié par chromatographie sur gel de silice en éluant avec un mélange 97/3 puis 95/5 de dichlorométhane et de méthanol.
On obtient 0,46 g de produit sous forme de solide beige.

### 1.3. 2-[1-(biphényl-4-ylméthyl)piperidin-4-yl]éthylcarbamate de 2-(méthylamino)-2-oxoéthyle

A une solution de 0,45 g (1,19 mmole) de 3-{2-[1-(biphényl-4-ylméthyl)pipéridin-4-yl]éthyl}-1,3-oxazolidine-2, 4-dione, obtenu à l'étape 1.2., dans 5 ml de méthanol, on ajoute 3 ml (5,97 mmoles) d'une solution de méthylamine (2M) dans le tétrahydrofurane. On poursuit l'agitation à température ambiante pendant 17 heures. Après concentration sous pression réduite, on purifie le résidu obtenu par chromatographie sur gel de silice en éluant avec un mélange 95/5 puis 90/10 de dichlorométhane et de méthanol. On obtient une pâte jaune que l'on cristallise dans le diisopropyléther.
On obtient 0,40 g de produit sous forme de solide jaune. LC-MS : M+H = 410
PF(°C) : 106-110°C
RMN¹H (CDCl₃) δ (ppm) : 1,2-1,50 (massif, 5H) ; 1,70 (m, 2H) ; 2,0 (large t, 2H) ; 2,90 (d, 3H) ; 3,0 (m, 2H) ; 3, 30 (q, 2H) ; 3,55 (s, 2H) ; 4,60 (s, 2H) ; 4,80 (large s, 1H) ; 6,15 (large s, 1H) ; 7,40 (m, 5H) ; 7,60 (m, 4H).

### Exemple 2 (composé N°52)

### (1-{2-[4-fluoro-2-(méthoxy)phényl]éthyl}pipéridin-4-yl)méthylcarbamate de 2-(méthylamino)-2-oxoéthyle

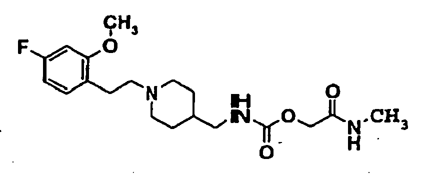

### 2.1. 4-{[(méthylsulfonyl)oxy]méthyl}pipéridine-1-carboxylate de 1,1-diméthyléthyle

A une solution de 10,08 g (46,81 mmoles) de 4-(hydroxyméthyl)pipéridine-1-carboxylate de 1,1-diméthyl éthyle et de 9,90 ml (70,21 mmoles) de triéthylamine dans 100 ml de dichlorométhane, refroidie à environ 0°C, on ajoute goutte à goutte, sous atmosphère inerte, une solution de 4 ml (51,49 mmoles) de chlorure de mésyle dans 10 ml de dichlorométhane. On enlève le bain et on poursuit l'agitation à température ambiante pendant 30 minutes. On ajoute de l'eau au milieu réactionnel, on sépare la phase aqueuse, on l'extrait une fois avec du dichlorométhane, on lave les phases organiques réunies avec de l'eau, on les sèche sur sulfate de sodium et on concentre le filtrat sous pression réduite.
On obtient ainsi 13,7 g de produit sous forme d'huile orange utilisé tel quel dans l'étape suivante.

### 2.2. 4-[(2,4-dioxo-1,3-oxazolidin-3-yl)méthyl]pipéridine-1-carboxylate de 1,1-diméthyléthyle

On porte au reflux pendant 24 heures une suspension de 13,60 g (46,36 mmoles) de 4-{[(méthylsulfonyl)oxy]méthyl} pipéridine-1-carboxylate de 1,1-diméthyléthyle, préparé à l'étape 2.1., de 9,37 g (92,72 mmoles) de 1,3-oxazolidin-2,4-dione et de 16,02 g (139,08 mmoles) de 1,1,3,3-tétraméthylguanidine, dans un mélange de 180 ml de tétrahydrofurane et de 30 ml de diméthylformamide. On laisse revenir à température ambiante et on concentre sous pression réduite. On reprend le résidu par du dichlorométhane et de l'eau, on sépare la phase aqueuse et on l'extrait deux fois avec du dichlorométhane. On lave les phases organiques réunies avec une solution aqueuse saturée en chlorure de sodium et on les sèche sur sulfate de sodium. Après évaporation du solvant, le résidu obtenu est purifié par chromatographie sur gel de silice en éluant avec un mélange 98/2 puis 95/5 de dichlorométhane et de méthanol.
On obtient 12,53 g de produit sous forme de solide orange-brun.

### 2.3. chlorhydrate de 3-(pipéridin-4-ylméthyl)-1,3-oxazolidine-2,4-dione

A une suspension de 12,51 g (41,95 mmoles) de 4-[(2,4-dioxo-1,3-oxazolidin-3-yl)méthyl]pipéridine-1-carboxylate de 1,1-diméthyléthyle, obtenu à l'étape 2.2., dans 65 ml de dioxane, on ajoute 38,10 ml (209,75 mmoles) d'une solution d'acide chlorhydrique (5N) dans l'isopropanol. On poursuit l'agitation à environ 60°C pendant 17 heures. On laisse revenir à température ambiante, On collecte le solide formé par filtration puis on le lave plusieurs fois par de l'éther et on le sèche sous vide à environ 70°C.
On obtient 8,41 g de produit sous forme de solide blanc.
PF (°C) : 195-200°C

### 2.4. 3-[(1-{2-[4-fluoro-2-(méthoxy)phényl]éthyl} pipéridin-4-yl)méthyl]-1,3-oxazolidine-2,4-dione

On utilise la méthode décrite dans L'exemple 1 (étape 1.2.). A partir de 0,40 g (1,70 mmole) de chlorhydrate de 3-(pipéridin-4-ylméthyl)-1,3-oxazolidine-2,4-dione, préparé à l'étape 2.3., de 0,423 g (1,70 mmole) de méthanesulfonate de 2-[4-fluoro-2-(méthyloxy)phényl] éthyle (EP1340761) et de 0,54 g (5,11 mmoles) de carbonate de sodium, on obtient après traitement 0,590 g de produit sous forme d'huile jaune visqueuse, utilisé tel quel dans l'étape suivante.

### 2.5. (1-{2- [4-fluoro-2-(méthoxy)phényl]éthyl}pipéridin-4-yl)méthylcarbamate de 2-(méthylamino)-2-oxoéthyle

On procède suivant le mode opératoire décrit dans l'exemple 1 (étape 1.3.). A partir de 0,58 g (1,66 mmole) de 3-[(1-{2-[4-fluoro-2-(méthoxy)phényl]éthyl}pipéridin-4-yl)méthyl]-1,3-oxazolidine-2,4-dione, préparé à l'étape 2.4., et de 8,28 ml (16,55 mmoles) d'une solution de méthylamine (2M) dans le tétrahydrofurane, et après chromatographie sur gel de silice en éluant avec un mélange 95/5/0,5 de dichlorométhane, de méthanol et d'ammoniaque à 28 %, suivie d'un lavage par le diisopropyléther, on obtient 0,315 g de produit sous forme de solide blanc.
LC-MS : M+H = 382
PF(°C) : 126-128°C
RMN¹H (DMSO) δ (ppm) : 1,10 (m, 2H) ; 1,35 (large s, 1H) ; 1,60 (large d, 2H) ; 1,85 (large t, 2H) ; 2,40 (m, 2H) ; 2,60 (m, 5H) ; 2,90 (m, 4H) ; 3,80 (s, 3H) ; 4,30 (s, 2H) ; 6,65 (td, 1H) ; 6,80 (dd, 1H) ; 7,1.5 (m, 2H) ; 7,70 (large s, 1H).

### Exemple 3 (composé N°68)

### (1-{2-[(2,4-dichlorophényl)-oxy]éthyl}pipéridin-4-yl)carbamate de 2-(méthylamino)-2-oxoéthyle

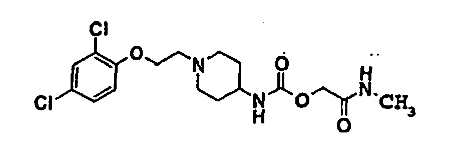

### 3.1. 4-[({[2-(éthyloxy)-2-oxoéthyl]oxy}carbonyl)amino] pipéridine-1-carboxylate de 1,1-diméthyléthyle

On chauffe au reflux pendant 30 heures, une suspension de 5,09 g (25,42 mmoles) de 4-aminopipéridine-1 -carboxylate de 1,1-diméthyléthyle et de 13,45 g (59,99 mmol e) de [(phényloxycarbonyl)oxy]acétate d'éthyle (J. Med. Chem., 1999, 42, 277-290) dans 300 ml de toluène.
On laisse revenir à température ambiante, on sépare l'insoluble par filtration et on concentre le filtrat sous pression réduite. On purifie le résidu ainsi obtenu par chromatographie sur gel de silice en éluant avec un mélange 30/70 d'acétate d'éthyle et de cyclohexane.
On obtient ainsi 6,62 g de produit sous forme d'huile jaune claire.

### 3.2. 4-[({[2-(méthylamino)-2-oxoéthyl]oxy}carbonyl)amino] pipéridine-1-carboxylate de 1,1-diméthyléthyle

On opère comme dans l'exemple 1 (étape 1.3.) . A partir de 6,33 g (19,16 mmole) de 4-[({[2-(éthyloxy)-2-oxoéthyl]oxy} carbonyl)amino]pipéridine-1-carboxylate de 1 ,1-diméthyl éthyle, préparé à l'étape 3.1., et de 47,90 ml (95,81 mmoles) d'une solution de méthylamine (2M) dans le tétrahydrofurane, on obtient 5,90 g de produit sous forme de pâte jaune collante.

### 3.3. chlorhydrate de pipéridin-4-ylcarbamate de 2-(méthylamino)-2-oxoéthyle

On utilise la méthode décrite dans l'exemple 2 (étape 2.3.). A partir de 5,90 g (18,71 mmoles) de 4-[({[2-(méthylamino)-2-oxoéthyl]oxy}carbonyl)amino]pipéridine-1-carboxylate de 1,1-diméthyléthyle, préparé à l'étape 3.2. et de 17 ml (93,53 mmoles) d'une solution d'acide chlorhydrique (5N) dans l'isopropanol, on obtient 3,83 g de chlorhydrate sous forme de solide blanc après lavage par le diisopropyléther et séchage sous vide à environ 70°C.
PF (°C) : 153°C

### 3.4. (1-{2-[(2,4-dichlorophényl)-oxy]éthyl}pipéridin-4-yl)carbamate de 2-(méthylamino)-2-oxoéthyle

On utilise la méthode décrite dans l'exemple 1 (étape 1.2.). A partir de 0,51 g (1,89 mmole) de chlorhydrate de pipéridin-4-ylcarbamate de 2-(méthylamino)-2-oxoéthyle, préparé à l'étape 3.3., de 0,50 g (1,99 mmole) de 1-[(2-bromoéthyl)oxy]-2,4-dichlorobenzene et de 0,60 g (5,68 mmoles) de carbonate de sodium, et après chromatographie sur gel de silice en éluant avec un mélange 94/6/0,6 puis 95/5/0,5 de dichlorométhane, de méthanol et d'ammoniaque à 28 %, suivie d'un lavage par le diisopropyléther, on obtient 0,44 g de produit sous forme de solide blanc. LC-MS : M+H = 404
PF(°C) : 115-119°C
RMN¹H (CDCl₃) δ (ppm) : 1,50 (m, 2H) ; 2,0 (large d, 2H) ; 2,35 (large t, 2H) ; 2,90 (d, 3H) ; 3,0 (m, 4H) ; 3,60 (m, 1H) ; 4,15 (t, 2H) ; 4,60 (s, 2H) ; 4, 75 (large d, 1H) ; 6, 15 (large s, 1H) ; 6,85 (d, 1H) ; 7,20 (dd, 1H) ; 7,40 (d, 1H) .

### Exemple 4 (composé N°4)

### chlorhydrate de {1-[(4-chlorophényl)méthyl]pipéridin-4-yl}méthylcarbamate de 2-(méthylamino)-2-oxoéthyle

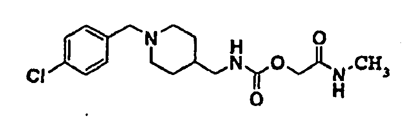

### 4.1. chlorhydrate de (pipéridin-4-yl)méthylcarbamate de 2-(méthylamino)-2-oxoéthyle

On agite à température ambiante pendant 15 heures une solution de 0,50 g (2,13 mmoles) de chlorhydrate de 3-(pipéridin-4-ylméthyl)-1,3-oxazolidine-2,4-dione, obtenu à l'étape 2.3. et de 5,30 ml (10,65 mmoles) d'une solution de méthylamine (2M) dans le tétrahydrofurane dans 10 ml de méthanol. Après concentration sous pression réduite, on traite le résidu obtenu avec une solution d'acide chlorhydrique (5N) dans l'isopropanol. On collecte le chlorhydrate obtenu par filtration, on le lave avec le diisopropyléther et on le sèche sous vide à environ 70°C. On obtient 0,49 g de poudre blanche.

### 4.2. chlorhydrate de {1-[(4-chlorophényl)méthyl] pipéridin-4-yl}méthylcarbamate de 2-(méthylamino)-2-oxoéthyle

On agite à température ambiante un mélange de 0,118 g (0,44 mmole) de chlorhydrate de (pipéridin-4-yl)méthylcarbamate de 2-(méthylamino)-2-oxoéthyle, de 0,283 g (1,33 mole) de triacétoxyborohydrure de sodium et de 0,626 g (4,45 mmoles) de 4-chlorobenzaldéhyde dans 5 ml d'une solution d'acide acétique à 1% dans le N,N-diméthylformamide. Après 24 heures d'agitation, on ajoute 2 g de résine acide DOWEX 50WX2 (Fluka) et on poursuit l'agitation à température ambiante pendant une heure. On filtre et on rince la résine avec 3 fois 5 ml de N,N-diméthylformamide, 3 fois 5 ml de dichlorométhane et 3 fois 5 ml de méthanol. On traite ensuite la résine pendant une heure à température ambiante avec 8 ml d'une solution (2M) d'ammoniac dans le méthanol. On filtre et on concentre le filtrat sous vide. On purifie le produit par chromatographie sur gel de silice en éluant avec un mélange 94/6 de dichlorométhane et de méthanol contenant 2% d'une solution aqueuse d'ammoniaque à 28%. On traite le résidu huileux obtenu avec 5 ml d'une solution d'acide chlorhydrique (0,1N) dans l'isopropanol. On concentre et on obtient 0,067 g de poudre blanche.
PF (°C) : 220-222°C
LC-MS : M+H = 354
RMN-¹H (DMSO-d₆ / D₂O) : δ (ppm) : 1,20 (m, 2H) ; 1,40 (m, 1H) ; 1,60 (m, 2H) ; 1,90 (t, 2H) ; 2,70 (s, 3H) ; 2, 75 (d, 2H) ; 2,90 (d, 2H) ; 3,40 (s, 2H) ; 4,30 (s, 2H) ; 7,95 (m, 4H).

Le tableau 1 qui suit illustre les structures chimiques et les propriétés physiques de quelques composés selon l'invention.
Dans ce tableau :
- dans la colonne « base ou sel », « base » signifie que le composé est sous forme de base libre, alors que « HCl » représente un composé sous forme de chlorhydrate et le rapport entre parenthèses est le rapport (acide:base),
- t-BuO, Me, Et et i-Pr, représentent respectivement des groupes tert-butoxy, méthyle, éthyle et isopropyle, et
- Ph représente un groupe phényle.

**Tableau 1**

| | | | | | | | | | | |
|---|---|---|---|---|---|---|---|---|---|---|
| 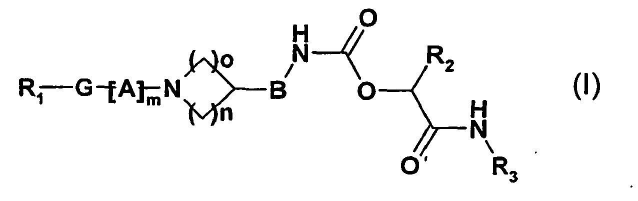 | | | | | | | | | | |

| **N°** | **R₁** | **G** | **[A]m** | **n** | **o** | **B** | **R₂** | **R₃** | **Base ou sel** | **PF(°C)** |
|---|---|---|---|---|---|---|---|---|---|---|
| 1. | 4-Cl-phényl | liaison | CH₂ | 2 | 2 | liaison | H | CH₃ | base | 144-146 |
| 2. | 4-Ph-phényl | liaison | CH₂ | 2 | 2 | liaison | H | CH₃ | base | 165-167 |
| 3. | 2-Cl-phényl | liaison | CH₂ | 2 | 2 | CH₂ | H | CH₃ | HCl (1/1) | 173-175 |
| 4. | 4-Cl-phényl | liaison | CH₂ | 2 | 2 | CH₂ | H | CH₃ | HCl (1/1) | 220-222 |
| 5. | 4-iPr-phényl | liaison | CH₂ | 2 | 2 | CH₂ | H | CH₃ | HCl (1/1) | 159-161 |
| 6. | 4-Ph-phényl | liaison | CH₂ | 2 | 2 | CH₂ | H | CH₃ | HCl (1/1) | 205-207 |
| 7. | [2-F,4-(4-F-phényl)]-phényl | liaison | CH₂ | 2 | 2 | CH₂ | H | CH₃ | base | 156-158 |
| 8. | [2-Cl,4-(4-F-phényl)]-phényl | liaison | CH₂ | 2 | 2 | CH₂ | H | CH₃ | HCl (1/1) | 163-169 |
| 9. | 3-(4-F-phényloxy)-phényl | liaison | CH₂ | 2 | 2 | CH₂ | H | CH₃ | HCl (1/1) | 163-165 |
| 10. | 4-[(3-F,4-Cl)-phényloxy]-phényl | liaison | CH₂ | 2 | 2 | CH₂ | H | CH₃ | base | 96-104 |
| 11. | naphtalèn-2-yl | liaison | CH₂ | 2 | 2 | CH₂ | H | CH₃ | HCl (1/1) | 156-157 |
| 12. | 4-Br-phényl | liaison | CH₂ | 2 | 2 | (CH₂)₂ | H | CH₃ | base | 107-111 |
| 13. | 3-CF₃-phényl | liaison | CH₂ | 2 | 2 | (CH₂)₂ | H | CH₃ | base | 90-92 |
| 14. | 4-CF₃-phényl | liaison | CH₂ | 2 | 2 | (CH₂)₂ | H | CH₃ | base | 116-120 |
| 15. | 3-CF₃O-phényl | liaison | CH₂ | 2 | 2 | (CH₂)₂ | H | CH₃ | base | 418* |
| 16. | 4-CF₃O-phényl | liaison | CH₂ | 2 | 2 | (CH₂)₂ | H | CH₃ | base | 109-111 |
| 17. | (2-Cl,3-Cl)-phényl | liaison | CH₂ | 2 | 2 | (CH₂)₂ | H | CH₃ | base | 114-116 |
| 18. | (2-Cl,4-Cl)-phényl | liaison | CH₂ | 2 | 2 | (CH₂)₂ | H | CH₃ | base | 117-119 |
| 19. | (2-Cl,5-Cl)-phényl | liaison | CH₂ | 2 | 2 | (CH₂)₂ | H | CH₃ | base | 125-127 |
| 20. | (3-Cl,4-Cl)-phényl | liaison | CH₂ | 2 | 2 | (CH₂)₂ | H | CH₃ | base | 101-105 |
| 21. | (3-Cl,5-Cl)-phényl | liaison | CH₂ | 2 | 2 | (CH₂)₂ | H | CH₃ | base | 124-126 |
| 22. | (2-Cl,5-F)-phényl | liaison | CH₂ | 2 | 2 | (CH₂)₂ | H | CH₃ | base | 102-104 |
| 23. | (2-F,3-Cl)-phényl | liaison | CH₂ | 2 | 2 | (CH₂)₂ | H | CH₃ | base | 104-106 |
| 24. | (3-Cl,5-CH₃)-phényl | liaison | CH₂ | 2 | 2 | (CH₂)₂ | H | CH₃ | base | 79-81 |
| 25. | 4-Ph-phényl | liaison | CH₂ | 2 | 2 | (CH₂)₂ | H | CH₃ | base | 106- 110 |
| 26. | [2-F,4-(4-F-phényl)]-phényl | liaison | CH₂ | 2 | 2 | (CH₂)₂ | H | CH₃ | base | 143-145 |
| 27. | [2-Cl,4-(4-F-phényl)]-phényl | liaison | CH₂ | 2 | 2 | (CH₂)₂ | H | CH₃ | base | 98-102 |
| 28. | 4-[(3-F,4-Cl)-phényloxy]-phényl | liaison | CH₂ | 2 | 2 | (CH₂)₂ | H | CH₃ | base | 109-111 |
| 29. | naphtalèn-1-yl | liaison | CH₂ | 2 | 2 | (CH₂)₂ | H | CH₃ | base | 86-88 |
| 30. | naphtalèn-2-yl | liaison | CH₂ | 2 | 2 | (CH₂)₂ | H | CH₃ | base | 87-93 |
| 31. | pyridin-2-yl | liaison | CH₂ | 2 | 2 | (CH₂)₂ | H | CH₃ | base | 104-106 |
| 32. | 3-CF₃-phényl | liaison | (CH₂)₂ | 2 | 2 | liaison | H | CH₃ | HCl (1/1) | 136-138 |
| 33. | 4-Cl-phényl | liaison | (CH₂)₂ | 2 | 2 | liaison | H | CH₃ | HCl (1/1) | 168-170 |
| 34. | 4-CN-phényl | liaison | (CH₂)₂ | 2 | 2 | liaison | H | CH₃ | base | 179-181 |
| 35. | (2-Cl,3-Cl)-phényl | liaison | (CH₂)₂ | 2 | 2 | liaison | H | CH₃ | base | 117-123 |
| 36. | (2-Cl,4-Cl)-phényl | liaison | (CH₂)₂ | 2 | 2 | liaison | H | CH₃ | base | 134-138 |
| 37. | (2-Cl,6-Cl)-phényl | liaison | (CH₂)₂ | 2 | 2 | liaison | H | CH₃ | base | 163-167 |
| 38. | (3-Cl,4-Cl)-phényl | liaison | (CH₂) ₂ | 2 | 2 | liaison | H | CH₃ | base | 130-132 |
| 39. | 4-Ph-phényl | liaison | (CH₂)₂ | 2 | 2 | liaison | H | CH₃ | base | 174-178 |
| 40. | 4-phényloxy-phényl | liaison | (CH₂)₂ | 2 | 2 | liaison | H | CH₃ | base | 144-146 |
| 41. | 4-F-naphtalèn-1yl- | liaison | (CH₂)₂ | 2 | 2 | liaison | H | CH₃ | HCl (1/1) | 185-187 |
| 42. | 4-F-naphtalèn-1-yl | liaison | (CH₂)₂ | 2 | 2 | liaison | H | CH₃ | base | 124-130 |
| 43. | naphtalèn-2-yl | liaison | (CH₂)₂ | 2 | 2 | liaison | H | CH₃ | HCl (1/1) | 198-202 |
| 44. | 3-Cl-phényl | liaison | (CH₂)₂ | 2 | 2 | CH₂ | H | CH₃ | base | 93-95 |
| 45. | 4-EtO-phényl | liaison | (CH₂)₂ | 2 | 2 | CH₂ | H | CH₃ | base | 134-136 |
| 46. | 4-Me₂N- phényl | liaison | (CH₂) ₂ | 2 | 2 | CH₂ | H | CH₃ | base | 130-132 |
| 47. | (2-Cl,3-Cl)-phényl | liaison | (CH₂)₂ | 2 | 2 | CH₂ | H | CH₃ | base | 81-87 |
| 48. | (2-Cl,4-Cl)-phényl | liaison | (CH₂)₂ | 2 | 2 | CH₂ | H | CH₃ | base | 125-127 |
| 49. | (2-Cl,4-Cl)-phényl | liaison | (CH₂)₂ | 2 | 2 | CH₂ | H | H | base | 115-119 |
| 50. | (2-Cl,6-Cl)-phényl | liaison | (CH₂)₂ | 2 | 2 | CH₂ | H | CH₃ | base | 144-148 |
| 51. | (3-Cl,4-Cl)-phényl | liaison | (CH₂)₂ | 2 | 2 | CH₂ | H | CH₃ | base | 123-127 |
| 52. | (2-MeO,4-F)-phényl | liaison | (CH₂)₂ | 2 | 2 | CH₂ | H | CH₃ | base | 126-128 |
| 53. | 4-phényloxy-phényl | liaison | (CH₂)₂ | 2 | 2 | CH₂ | H | CH₃ | base | 120-124 |
| 54. | naphtalèn-1-yl | liaison | (CH₂)₂ | 2 | 2 | CH₂ | H | CH₃ | HCl (1/1) | 148-150 |
| 55. | 4-F-naphtalèn-1-yl | liaison | (CH₂)₂ | 2 | 2 | CH₂ | H | CH₃ | base | 120-124 |
| 56. | naphtalèn-2-yl | liaison | (CH₂)₂ | 2 | 2 | CH₂ | H | CH₃ | base | 163-166 |
| 57. | 2-Cl-phényl | liaison | (CH₂)₂ | 2 | 2 | (CH₂)₂ | H | CH₃ | HCl (1/1) | 121-123 |
| 58. | 4-F-phényl | liaison | (CH₂)₂ | 2 | 2 | (CH₂)₂ | H | CH₃ | base | 122-124 |
| 59. | 4-EtO-phényl | liaison | (CH₂)₂ | 2 | 2 | (CH₂)₂ | H | CH₃ | base | 118-120 |
| 60. | (2-Cl,6-F)-phényl | liaison | (CH₂)₂ | 2 | 2 | (CH₂)₂ | H | CH₃ | base | 88-90 |
| 61. | 4-Cl-phényl | liaison | (CH₂)₃ | 2 | 2 | liaison | H | CH₃ | HCl (1/1) | 132-134 |
| 62. | 4-MeO-phényl | liaison | (CH₂)₃ | 2 | 2 | liaison | H | CH₃ | HCl (1/1) | 175-177 |
| 63. | 4-MeO-phényl | liaison | (CH₂)₃ | 2 | 2 | CH₂ | H | CH₃ | HCl (1/1) | 155-157 |
| 64. | 4-MeO-phényl | liaison | (CH₂)₃ | 2 | 2 | (CH₂) ₂ | H | CH₃ | HCl (1/1) | 118-120 |
| 65. | 4-F-phényl | O | (CH₂)₂ | 2 | 2 | liaison | H | CH₃ | base | 135-137 |
| 66. | 4-Cl-phényl | O | (CH₂)₂ | 2 | 2 | liaison | H | CH₃ | base | 141-145 |
| 67. | (2-Cl,3-Cl)-phényl | O | (CH₂)₂ | 2 | 2 | liaison | H | CH₃ | base | 128-130 |
| 68. | (2-Cl,4-Cl)-phényl | O | (CH₂)₂ | 2 | 2 | liaison | H | CH₃ | base | 115-119 |
| 69. | (2-Cl,4-Cl)-phényl | O | CH(CH₃)CH₂ | 2 | 2 | liaison | H | CH₃ | HCl (1/1) | 141-143 |
| 70. | (3-Cl,4-Cl)-phényl | O | (CH₂)₂ | 2 | 2 | liaison | H | CH₃ | base | 146-148 |
| 71. | 4-Cl-naphtalèn-1-yl | O | (CH₂)₂ | 2 | 2 | liaison | H | CH₃ | base | 136-140 |
| 72. | 4-Cl-naphtalèn-1-yl | O | (CH₂)₂ | 2 | 2 | liaison | H | H | base | 141-143 |
| 73. | quinolin-5-yl | O | (CH₂)₂ | 2 | 2 | liaison | H | CH₃ | base | 140-142 |
| 74. | isoquinolin-5-yl | O | (CH₂)₂ | 2 | 2 | liaison | H | CH₃ | base | 149-153 |
| 75. | 4-Cl-phényl | O | (CH₂)₂ | 2 | 2 | CH₂ | H | CH₃ | base | 115-119 |
| 76. | (2-Cl,3-Cl)-phényl | O | (CH₂)₂ | 2 | 2 | CH₂ | H | CH₃ | base | 117-119 |
| 77. | (2-Cl,4-Cl)-phényl | O | (CH₂)₂ | 2 | 2 | CH₂ | H | CH₃ | HCl (1/1) | 137-139 |
| 78. | (2-Cl,4-Cl)-phényl | O | CH(CH₃)CH₂ | 2 | 2 | CH₂ | H | CH₃ | HCl (1/1) | 194-196 |
| 79. | (3-Cl,4-Cl)-phényl | O | (CH₂)₂ | 2 | 2 | CH₂ | H | CH₃ | base | 98-100 |
| 80. | 4-Cl-naphtalèn-1-yl | O | (CH₂)₂ | 2 | 2 | CH₂ | H | CH₃ | base | 116-120 |
| 81. | quinolin-5-yl | O | (CH₂)₂ | 2 | 2 | CH₂ | H | CH₃ | HCl (1/1) | 220-224 |
| 82. | isoquinolin-5-yl | O | (CH₂)₂ | 2 | 2 | CH₂ | H | CH₃ | HCl (1/1) | 74-78 |
| 83. | 4-F-phényl | O | (CH₂)₂ | 2 | 2 | (CH₂)₂ | H | CH₃ | base | 125-129 |
| 84. | 4-Cl-phényl | O | (CH₂)₂ | 2 | 2 | (CH₂)₂ | H | CH₃ | base | 109-113 |
| 85. | 4-Cl-phényl | O | (CH₂)₃ | 2 | 2 | liaison | H | CH₃ | base | 133-137 |
| 86. | 4-F-phényl | O | (CH₂)₂ | 1 | 2 | CH₂ | H | CH₃ | HCl (1/1) | 354* |
| 87. | 4-Cl-phényl | O | (CH₂)₂ | 1 | 2 | CH₂ | H | CH₃ | base | 67-69 |
| 88. | 3-(4-F-phényloxyp)-phényl | liaison | CH₂ | 2 | 2 | liaison | H | CH₃ | base | 107-109 |
| 89. | 5-(4-Cl-phényl)-isoxazol-3-yl | liaison | (CH₂)₃ | 2 | 2 | liaison | H | CH₃ | base | 151-153 |
| 90. | 3-CF₃O-phényl | liaison | CH₂ | 2 | 2 | CH₂ | H | CH₃ | base | 73-75 |
| 91. | 4-CF₃O-phényl | liaison | CH₂ | 2 | 2 | CH₂ | H | CH₃ | base | 104-106 |
| 92. | 3-(pyrimidin-2-yloxy)-phényl | liaison | CH₂ | 2 | 2 | CH₂ | H | CH₃ | HCl (1/1) | 196-200 |
| 93. | 3-(4-Cl-phényl)-5-isoxazol-5-yl | liaison | CH₂ | 2 | 2 | CH₂ | H | CH₃ | base | 148-150 |
| 94. | 5-(4-Cl-phényl)-1,3-oxazol-2-yl | liaison | CH₂ | 2 | 2 | CH₂ | H | CH₃ | base | 143-145 |
| 95. | 4-(4-CF₃-phényl)-1,3-thiazol-2-yl | liaison | CH₂ | 2 | 2 | CH₂ | H | CH₃ | base | 172-174 |
| 96. | 5-(4-Cl-phényl)-isoxazol-3-yl | liaison | (CH₂)₂ | 2 | 2 | CH₂ | H | CH₃ | base | 165-167 |
| 97. | 3-(4-Cl-phényl)-isoxazol-5-yl | liaison | (CH₂)₂ | 2 | 2 | CH₂ | H | CH₃ | base | 146-148 |
| 98. | 3-(4-Cl-phényl)-isoxazol-5-yl | liaison | (CH₂)₃ | 2 | 2 | CH₂ | H | CH₃ | base | 134-136 |
| 99. | 5-(4-Cl-phényl)-isoxazol-3-yl | liaison | (CH₂)₃ | 2 | 2 | CH₂ | H | CH₃ | base | 160-162 |
| 100. | (2-Cl,4-F)-phényl | liaison | CH₂ | 2 | 2 | (CH₂)₂ | H | CH₃ | base | 108-110 |
| 101. | (3-Cl,4-F)-phényl | liaison | CH₂ | 2 | 2 | (CH₂)₂ | H | CH₃ | base | 112-114 |
| 102. | (3-CN,5-F)-phényl | liaison | CH₂ | 2 | 2 | (CH₂)₂ | H | CH₃ | base | 132-134 |
| 103. | 3-(4-F-phényloxy)-phényl | liaison | CH₂ | 2 | 2 | (CH₂)₂ | H | CH₃ | base | 79-81 |
| 104. | 3-(4-Cl-phényl)- isoxazol-5-yl | liaison | CH₂ | 2 | 2 | (CH₂)₂ | H | CH₃ | base | 169-171 |
| 105. | 5-(4-Cl-phényl)-1,3- oxazol-2-yl | liaison | CH₂ | 2 | 2 | (CH₂)₂ | H | CH₃ | base | 124-126 |
| 106. | 4-(4-CF₃-phényl)-1,3-thiazol-2-yl | liaison | CH₂ | 2 | 2 | (CH₂)₂ | H | CH₃ | base | 161-163 |
| 107. | (2-Cl,4-F)-phényl | liaison | CH(CH₃) | 2 | 2 | CH₂ | H | CH₃ | HCl (1/1) | 386* |
| 108. | 3-(4-Cl-phényloxy)-phényl | liaison | CH(CH₃) | 2 | 2 | CH₂ | H | CH₃ | HCl (1/1) | 130-134 |
| 109. | [2-Cl,3-(4-Cl-phényloxy)]-phényl | liaison | CH(CH₃) | 2 | 2 | CH₂ | H | CH₃ | base | 115-119 |
| 110. | 3-CF₃O- phényl | liaison | CH(CH₃) | 2 | 2 | (CH₂)₂ | H | CH₃ | HCl (1/1) | 169-171 |
| 111. | (2-Cl,4-F)-phényl | liaison | CH(CH₃) | 2 | 2 | (CH₂)₂ | H | CH₃ | HCl (1/1) | 400* |
| 112. | 4-Cl-phényl | liaison | C≡C-(CH₂)₂ | 2 | 2 | liaison | H | CH₃ | base | 189-191 |
| 113. | 4-Cl-phényl | liaison | C≡C-(CH₂)₃ | 2 | 2 | liaison | H | CH₃ | base | 137-139 |
| 114. | (2-Cl,5-Cl) phényl | liaison | C≡C-(CH₂)₃ | 2 | 2 | liaison | H | CH₃ | base | 426* |
| 115. | (2-Cl,4-F)-phényl | liaison | C≡C-(CH₂)₃ | 2 | 2 | liaison | H | CH₃ | base | 410* |
| 116. | 4-Cl-phényl | liaison | C≡C-(CH₂)₂ | 2 | 2 | CH₂ | H | CH₃ | base | 151-153 |
| 117. | (2-Cl,3-Cl)-phényl | O | (CH₂)₂ | 1 | 1 | liaison | H | CH₃ | base | 138-140 |
| 118. | (2-F,4-Cl)-phényl | liaison | C≡C-(CH₂)₂ | 2 | 2 | CH₂ | H | CH₃ | base | 144-146 |
| 119. | (2-Cl,5-Cl)-phényl, | liaison | C≡C-(CH₂)₂ | 2 | 2 | CH₂ | H | CH₃ | base | 128-130 |
| 120. | 3-(4-Cl-phényl)-isoxazol-5-yl | liaison | (CH₂)₂ | 2 | 2 | (CH₂)₂ | H | CH₃ | base | 135-137 |
| 121. | 3-*t*-BuO-phényl | liaison | CH₂ | 2 | 2 | CH₂ | H | CH₃ | base | 392* |
| 122. | 3-*t*-BuO- phényl | liaison | CH₂ | 2 | 2 | (CH₂)₂ | H | CH₃ | base | 66-70 |

| | | | | | | | | | | |
|---|---|---|---|---|---|---|---|---|---|---|
| * M+H | | | | | | | | | | |

Les composés de l'invention ont fait l'objet d'essais pharmacologiques permettant de déterminer leur effet inhibiteur de l'enzyme FAAH (Fatty Acid amido Hydrolase).

L'activité inhibitrice a été mise en évidence dans un test radioenzymatique basé sur la mesure du produit d'hydrolyse (éthanolamine [1-³H] ) de l'anandamide [éthanolamine 1-³H] par la FAAH (Life Sciences (1995), 56, 1999-2005 et Journal of Pharmacology and Experimental Therapeutics (1997), 283, 729-734). Ainsi, les cerveaux de souris (moins le cervelet) sont prélevés et conservés à -80°C. Les homogénats membranaires sont préparés extemporanément par homogénéisation des tissus au Polytron dans un tampon Tris-HCl 10mM (pH 8,0) contenant 150 mM NaCl et 1 mM EDTA. La réaction enzymatique est ensuite conduite dans 70 µl de tampon contenant de l'albumine de sérum bovin sans acides gras (1 mg/ml). Sont ajoutés successivement les composés testés à différentes concentrations, l'anandamide [éthanolamine 1-³H] (activité spécifique de 15-20 Ci/mmol) diluée à 10 µM avec de l'anandamide froide et la préparation membranaire (400 µg tissu congelé par essai). Après 15 minutes à 25°C, la réaction enzymatique est arrêtée par addition de 140 µL de chloroforme/méthanol (2 :1). Le mélange est agité 10 minutes puis centrifugé pendant 15 minutes à 3500g. Un aliquot (30 µL) de la phase aqueuse contenant l'éthanolamine [1-³H] est comptée par scintillation liquide. Dans ces conditions, les composés les plus actifs de l'invention présentent des CI₅₀ (concentration inhibant de 50% l'activité enzymatique contrôle de la FAAH) comprises entre 0,001 et 1 µM.
Le tableau 2 ci-dessous présente les CI₅₀ de quelques composés selon l'invention.

**Tableau 2**

| Composé N° | CI₅₀ |
|---|---|
| 25 | 0,225 µM |
| 77 | 0,049 µM |

Il apparaît donc que les composés selon l'invention ont une activité inhibitrice sur l'enzyme FAAH.

L'activité *in vivo* des composés de l'invention a été évaluée dans un test d'analgésie.
Ainsi, l'administration intrapéritonéale (i.p.) de PBQ (phénylbenzoquinone, 2 mg/kg dans une solution de chlorure de sodium à 0,9% contenant 5%, d'éthanol) chez des souris mâles OF1 de 25 à 30 g, provoque des étirements abdominaux, en moyenne 30 torsions ou contractions pendant la période de 5 à 15 minutes après injection. Les composés testés sont administrés par voie orale (p.o.) ou par voie intrapéritonéale (i.p.) en suspension dans du Tween 80 à 0,5%, 60 minutes ou 120 minutes avant l'administration de PBQ. Dans ces conditions, les composés les plus puissants de l'invention réduisent de 35 à 70 % le nombre d'étirements induits par le PBQ, dans une gamme de doses comprise entre 1 et 30 mg/kg.
Par exemple, le composé n° 26 du tableau réduit de 56% le nombre d'étirements induits par le PBQ, à la dose de 10 mg/kg p.o. à 120 minutes.

L'enzyme FAAH (Chemistry and Physics of Lipids, (2000), 108, 107-121) catalyse l'hydrolyse des dérivés endogènes d'amides et d'esters de différents acides gras tels que la *N*-arachidonoyléthanolamine (anandamide), la *N*-palmitoyléthanolamine, la *N*-oléoyléthanolamine, l'oléamide ou le 2-arachidonoylglycérol. Ces dérivés exercent différentes activités pharmacologiques en interagissant, entre autres, avec les récepteurs cannabinoïdes et vanilloïdes.
Les composés de l'invention, bloquent cette voie de dégradation et augmentent le taux tissulaire de ces substances endogènes. Ils peuvent être utilisés à ce titre dans la prévention et le traitement des pathologies dans lesquelles les cannabinoides endogènes et/ ou tous autres substrats métabolisés par l'enzyme FAAH, sont impliqués.
On peut par exemple citer les maladies et les affections suivantes :
La douleur notamment les douleurs aiguës ou chroniques de type neurogène : migraine, douleurs neuropathiques incluant les formes associées au virus de l'herpès et au diabète ;
les douleurs aiguës ou chroniques associées aux maladies inflammatoires : arthrite, arthrite rhumatoïde, ostéoarthrite, spondylite, goutte, vascularite, maladie de Crohn, syndrome du colon irritable ;
les douleurs aiguës ou chroniques périphériques ;
les vertiges, les vomissements, les nausées en particulier celles consécutives à une chimiothérapie ;
les troubles du comportement alimentaire en particulier les anorexies et cachexies de diverses natures ;
les pathologies neurologiques et psychiatrique tremblements, dyskinésie, dystonies, spasticité, comportements compulsifs et obsessionnels, syndrome de Tourette, toutes les formes de dépression et d'anxiété de toute nature et origine, troubles de l'humeur, psychoses ;
les maladies neuro-dégénératives aiguës et chroniques : maladie de Parkinson, maladie d'Alzheimer, démence sénile, chorée de Huntington, lésions liées à l'ischémie cérébrale et aux traumatismes crâniens et médullaires ;
l'épilepsie ;
les troubles du sommeil incluant les apnées du sommeil ;
les maladies cardiovasculaires en particulier hypertension, arythmies cardiaques, artériosclérose, crise cardiaque, ischémies cardiaques ;
l'ischémie rénale ;
les cancers : tumeurs bénignes de la peau, papillomes et tumeurs cérébrales, tumeurs de la prostate, tumeurs cérébrales (glioblastomes, médullo-épithéliomes, médulloblastomes, neuroblastomes, tumeurs d'origine embryonnaires, astrocytomes, astroblastomes, épendyomes, oligodendrogliomes, tumeur du plexus, neuroepithéliomes, tumeur de l'épiphyse, épendymoblastomes, méningiomes malins, sarcomatoses, mélanomes malins, schwénnomes) ;
les désordres du système immunitaire, notamment les maladies auto-immunes : psoriasis, lupus érythémateux, maladies du tissu conjonctif ou connectivites, syndrome de Sjögren's, spondylarthrite ankylosante, spondylarthrite indifférenciée, maladie de Behcet's, anémies auto-immunes hémolytiques, sclérose en plaques, sclérose latérale amyotrophique, amyloses, rejet de greffes, maladies affectant la lignée plasmocytaire ;
les maladies allergiques : l'hypersensibilité immédiate ou retardée, rhinites ou conjonctivites allergiques, dermatites de contact ;
les maladies infectieuses parasitaires , virales ou bactériennes : SIDA, méningites ; les maladies inflammatoires, notamment les maladies articulaires: arthrite, arthrite rhumatoïde, ostéoarthrite, spondylite, goutte, vascularite, maladie de Crohn, syndrome du colon irritable; l'ostéoporose; les affections oculaire: hypertension oculaire, glaucome;
les affections pulmonaires: maladies des voies respiratoires, bronchospasmes, toux, asthme, bronchite chronique, obstruction chronique des voies respiratoires, emphysème;
les maladies gastro-intestinales: syndrome du colon irritable, désordres inflammatoires intestinaux, ulcères, diarrhées ;
l'incontinence urinaire et l'inflammation vésicale.

L'utilisation d'un composé de formule (I), à l'état de base, de sel, d'hydrate ou de solvat pharmaceutiquement acceptable, pour la préparation d'un médicament destiné à traiter les pathologies ci-dessus mentionnées fait partie intégrante de l'invention.

L'invention a également pour objet des médicaments qui comprennent un composé de formule (I), ou un sel, ou encore un hydrate ou un solvat pharmaceutiquement acceptable du composé de formule (I). Ces médicaments trouvent leur emploi en thérapeutique, notamment dans le traitement des pathologies ci-dessus mentionnées.

Selon un autre de ses aspects, la présente invention concerne des compositions pharmaceutiques renfermant en tant que principe actif, au moins un composé selon l'invention. Ces compositions pharmaceutiques contiennent une dose efficace d'un composé selon l'invention, ou un sel, ou un hydrate, ou un solvat pharmaceutiquement acceptable dudit composé, et éventuellement un ou plusieurs excipients pharmaceutiquement acceptables.

Lesdits excipients sont choisis selon la forme pharmaceutique et le mode d'administration souhaité, parmi les excipients habituels qui sont connus de l'homme du métier.

Dans les compositions pharmaceutiques de la présente invention pour l'administration orale, sublinguale, sous-cutanée, intramusculaire, intra-veineuse, topique, locale, intrathécale, intranasale, transdermique, pulmonaire, oculaire ou rectale, le principe actif de formule (I) ci-dessus, ou son sel, solvat ou hydrate éventuel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules, les chewing-gums et les solutions ou suspensions orales, les formes d'administration sublinguale, buccale, intratrachéale, intraoculaire, intranasale, par inhalation, les formes d'administration sous-cutanée, intramusculaire ou intraveineuse et les formes d'administration rectale ou vaginale. Pour l'application topique, on peut utiliser les composés selon l'invention dans des crèmes, pommades ou lotions.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscaramellose sodique | 6,0 mg |
| Amidon de maïs | 15,0 mg |
| Hydroxypropyl-méthylcellulose | 2,25 mg |
| Stéarate de magnésium | 3,0 mg |

Lesdites formes unitaires sont dosées pour permettre une administration journalière de 0,01 à 20 mg de principe actif par kg de poids corporel, selon la forme galénique.

Il peut y avoir des cas particuliers où des dosages plus élevés ou plus faibles sont appropriés, de tels dosages appartiennent également à l'invention. Selon la pratique habituelle, le dosage approprié à chaque patient est déterminé par le médecin selon le mode d'administration, le poids et la réponse dudit patient.

## Revendications

1. Composé répondant à la formule (I) 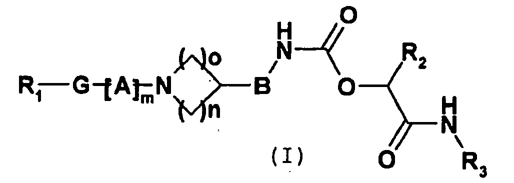 dans laquelle
m représente un nombre entier allant de 1 à 4 ;
n représente un nombre entier égal à 1, 2 ou 3 ;
o représente un nombre entier égal à 1 ou 2;
A est choisi parmi un ou plusieurs groupes X, Y et/ou Z ;
X représente un groupe méthylène éventuellement substitué par un ou deux groupes C₁₋₆-alkyle, C₃₋₇-cycloalkyle ou C₃₋₇-cycloalkyl-C₁₋₃-alkylène;
Y représente soit un groupe C₂-alcènylène éventuellement substitué par un ou deux groupes C₁₋₆ alkyle, C₃₋₇-cycloalkyle ou C₃₋₇-cycloalkyl-C₁₋₃-alkylène; soit un groupe C₂-alcynylène ;
Z représente un groupe de formule : 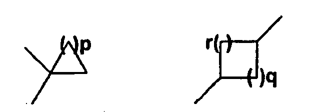
p représente un nombre entier allant de 1 à 5 ;
q et r représentent des nombres entiers et sont définis tels que r+q soit un nombre allant de 1 à 5 ;
B représente une liaison covalente ou un groupe C₁₋₆-alkylène ;
G représente une liaison covalente, un atome d'oxygène ou de soufre ou un groupe -CH(OH)-, CO, SO ou SO₂ ;
R₁ représente un groupe R₄ éventuellement substitué par un ou plusieurs groupes R₅ et/ou R₆ ;
R₄ représente un groupe choisi parmi un furanyle, pyrrolyle, thiènyle, oxazolyle, isoxazolyle, thiazolyle, isothiazolyle, imidazolyle, pyrazolyle, thiadiazolyle, isothiadiazolyle, oxadiazolyle, thiadiazolyle, triazolyle, tétrazolyle, phényle, pyridinyle, pyridazinyle, pyrimidinyle, pyrazinyle, triazinyle, naphtalènyle, quinolinyle, tétrahydroquinolinyle, isoquinolinyle, tétrahydroisoquinolinyle, phtalazinyle, quinazolinyle, quinoxalinyle, cinnolinyle, naphthyridinyle, imidazopyrimidinyle, thiènopyrimidinyle, benzofuranyle, dihydrobenzofuranyle, benzothiènyle, dihydrobenzothiènyle, benzimidazolyle, benzoxazolyle, benzisoxazolyle, benzothiazolyle, benzisothiazolyle, indolyle, isoindolyle, indazolyle, pyrrolopyridinyle, furopyridinyle, dihydrofuropyridinyle, thiènopyridinyle, dihydrothiénopyridinyle, imidazopyridinyle, pyrazolopyridinyle, oxazolopyridinyle, isoxazolopyridinyle, thiazolopyridinyle ;
R₅ représente un atome d'halogène, un groupe cyano, nitro, hydroxyle, C₁₋₆-alkyle, C₁₋₆-alcoxy, C₁₋₆-thioalkyle, C₁₋₆-fluoroalkyle, C₁₋₆-fluoroalcoxy, C₁₋₆-fluorothioalkyle, C₃₋₇-cycloalkyle, C₃₋₇-cycloalkyle-C₁₋₃-alkylène, NR₇R₈, NR₇COR₈, NR₇CO₂R₈, NR₇SO₂R₈, COR₇, CO₂R₇, CONR₇R₈, SO₂R₇, SO₂NR₇R₈ ou -O-(C₁₋₃-alkylène)-O- ;
R₆ représente un groupe phényle, phényloxy, benzyloxy, pyridinyle, pyrazinyle, pyridazinyle, pyrimidinyle ou pyrimidinyloxy; le ou les groupes R₆ pouvant être substitués par un ou plusieurs groupes R₅ identiques ou différents l'un de l'autre ;
R₇ et R₈ représentent indépendamment l'un de l'autre un atome d'hydrogène ou un groupe C₁₋₆-alkyle, ou forment avec le ou les atomes qui les portent un cycle choisi parmi un cycle azétidine, pyrrolidine, pipéridine, morpholine, thiomorpholine, azépine ou pipérazine, ce cycle étant éventuellement substitué par un groupe C₁₋₆-alkyle ou benzyle ;
R₂ représente un atome d'hydrogène ou un groupe C₁₋₆-alkyle ;
R₃ représente un atome d'hydrogène ou un groupe C₁₋₆-alkyle, C₃₋₇-cycloalkyle, C₃₋₇-cycloalkyle-C₁₋₃-alkylène ;
à l'état de base, de sel d'addition à un acide, d'hydrate ou de solvat.

2. Composé de formule (I) selon la revendication 1,
**caractérisé en ce que :**
m représente un nombre entier allant de 1 à 4 ;
n représente un nombre entier égal à 1 ou 2 ;
o représente un nombre entier égal à 1 ou 2;
A est choisi parmi un ou plusieurs groupes X et/ou Y ;
X représente un groupe méthylène éventuellement substitué par un ou deux groupes C₁₋₆-alkyle;
Y représente un groupe C₂-alcynylène ;
B représente une liaison covalente ou un groupe C₁₋₆-alkylène ;
G représente une liaison covalente ou un atome d'oxygène ;
R₁ représente un groupe R₄ éventuellement substitué par un ou plusieurs groupes R₅ et/ou R₆ ;
R₄ représente un groupe choisi parmi un oxazolyle, isoxazolyle, thiazolyle, phényle, pyridinyle, naphtalènyle, quinolinyle, isoquinolinyle ;
R₅ représente un atome d'halogène, un groupe cyano, NR₇R₈, un groupe C₁₋₆-alkyle, C₁₋₆-alcoxy, C₁₋₆-fluoroalkyle ou C₁₋₆-fluoroalcoxy ;
R₆ représente un groupe phényle, phényloxy ou pyrimidinyloxy; le ou les groupes R₆ pouvant être substitués par un ou plusieurs groupes R₅ identiques ou différents l'un de l'autre ;
R₇ et R₈ représentent indépendamment l'un de l'autre un groupe C₁₋₆-alkyle ;
R₂ représente un atome d'hydrogène ou un groupe C₁₋₆-alkyle ;
R₃ représente un atome d'hydrogène ou un groupe C₁₋₆-alkyle, C₃₋₇-cycloalkyle, C₃₋₇-cycloalkyle-C₁₋₃alkylène ;
à l'état de base, de sel d'addition à un acide, d'hydrate ou de solvat.

3. Composé de formule (I) selon la revendication 1 ou 2, **caractérisé en ce que** :
m représente un nombre entier égal à 1 ou 2 ;
à l'état de base, de sel d'addition à un acide, d'hydrate ou de solvat.

4. Composé de formule (I) selon l'une quelconque des revendications 1 à 3, **caractérisé en ce que** :
n est égal à 2 ;
o est égal à 2 ;
à l'état de base, de sel d'addition à un acide, d'hydrate ou de solvat.

5. Composé de formule (I) selon l'une quelconque des revendications 1 à 4, **caractérisé en ce que** :
R₁ représente un groupe R₄ éventuellement substitué par un ou plusieurs groupes R₅ et/ou R₆ ;
R₄ représente un groupe choisi parmi un oxazolyle, isoxazolyle, phényle ou naphtalènyle ;
R₅ représente un atome d'halogène, un groupe cyano, NR₇R₈, un groupe C₁₋₆-alkyle, C₁₋₆-alcoxy, C₁₋₆-fluoroalkyle ou C₁₋₆-fluoroalcoxy ;
R₆ représente un groupe phényle, phényloxy ou pyrimidinyloxy; le ou les groupes R₆ pouvant être substitués par un ou plusieurs groupes R₅ identiques ou différents l'un de l'autre ;
R₇ et R₈ représentent indépendamment l'un de l'autre un groupe C₁₋₆-alkyle ;
à l'état de base, de sel d'addition à un acide, d'hydrate ou de solvat.

6. Composé de formule (I) selon l'une quelconque des revendications 1 à 5, **caractérisé en ce que** :
R₂ représente un atome d'hydrogène ;
R₃ représente un atome d'hydrogène ou un groupe C₁₋₆-alkyle ;
à l'état de base, de sel d'addition à un acide, d'hydrate ou de solvat.

7. Composé de formule (I) selon la revendication 1 choisi parmi les composés suivants :
- {1-[(3,4'-difluorobiphényl-4-yl)méthyl]pipéridin-4-yl}méthylcarbamate de 2-(méthylamino)-2-oxoéthyle ;
- {1-[(3-chloro-4'-fluorobiphényl-4-yl)méthyl]pipéridin-4-yl}méthylcarbamate de 2-(méthylamino)-2-oxoéthyle ;
- {1-[3-(4-fluorophénoxy)benzyl]pipéridin-4-yl}méthylcarbamate de 2-(méthylamino)-2-oxoéthyle ;
- {1-[4-(4-chloro-3-fluorophénoxy)benzyl]pipéridin-4-yl}méthylcarbamate de 2-(méthylamino)-2-oxoéthyle ;
- 2-{1-[3-(trifluorométhoxy)benzyl]pipéridin-4-yl}éthylcarbamate de 2-(méthylamino)-2-oxoéthyle ;
- 2-{1-[4-(trifluorométhoxy)benzyl]pipéridin-4-yl}éthylcarbamate de 2-(méthylamino)-2-oxoéthyle ;
- 2-[1-(3-*tert*-butoxybenzyl)pipéridin-4-yl]éthylcarbamate de 2-(méthylamino)-2-oxoéthyle ;
- 2-[1-(3-*tert*-butoxybenzyl)pipéridin-4-yl]méthylcarbamate de 2-(méthylamino)-2-oxoéthyle ;
- 2-[1-(2,4-dichlorobenzyl)pipéridin-4-yl]éthylcarbamate de 2-(méthylamino)-2-oxoéthyle ;
- 2-[1-(2,5-dichlorobenzyl)pipéridin-4-yl]éthylcarbamate de 2-(méthylamino)-2-oxoéthyle ;
- 2-[1-(3,5-dichlorobenzyl)pipéridin-4-yl]éthylcarbamate de 2-(méthylamino)-2-oxoéthyle ;
- 2-[1-(2-chloro-5-fluorobenzyl)pipéridin-4-yl]éthylcarbamate de 2-(méthylamino)-2-oxoéthyle ;
- 2-[1-(3-chloro-2-fluorobenzyl)pipéridin-4-yl]éthylcarbamate de 2-(méthylamino)-2-oxoéthyle ;
- 2-[1-(3-chloro-5-méthylbenzyl)pipéridin-4-yl]éthylcarbamate de 2-(méthylamino)-2-oxoéthyle ;
- 2-{1-[(3,4'-difluorobiphényl-4-yl)méthyl]pipéridin-4-yl}éthylcarbamate de 2-(méthylamino)-2-oxoéthyle ;
- 2-{1-[(3-chloro-4'-fluorobiphényl-4-yl)méthyl]pipéridin-4-yl}éthylcarbamate de 2-(méthylamino)-2-oxoéthyle ;
- 2-{1-[4-(4-chloro-3-fluorophénoxy)benzyl]pipéridin-4-yl}éthylcarbamate de 2-(méthylamino)-2-oxoéthyle ;
- {1-[3-(4-fluorophénoxy)benzyl]pipéridin-4-yl}carbamate de 2-(méthylamino)-2-oxoéthyle ;
- {1-[3-(trifluorométhoxy)benzyl]pipéridin-4-yl}méthylcarbamate de 2-(méthylamino)-2-oxoéthyle ;
- {1-[4-(trifluorométhoxy)benzyl]pipéridin-4-yl}méthylcarbamate de 2-(méthylamino)-2-oxoéthyle ;
- {1-[3-(pyrimidin-2-yloxy)benzyl]pipéridin-4-yl}méthylcarbamate de 2-(méthylamino)-2-oxoéthyle ;
- 2-[1-(2-chloro-4- fluorobenzyl)pipéridin-4-yl]éthylcarbamate de 2-(méthylamino)-2-oxoéthyle ;
- 2-[1-(3-chloro-4-fluorobenzyl)pipéridin-4-yl]éthylcarbamate de 2-(méthylamino)-2-oxoéthyle ;
- 2-[1-(3-cyano-5-fluorobenzyl)pipéridin-4-yl]éthylcarbamate de 2-(méthylamino)-2-oxoéthyle ;
- 2-{1-[3-(4-fluorophénoxy)benzyl]pipéridin-4-yl}éthylcarbamate de 2-(méthylamino)-2-oxoéthyle ;
- (1-{[3-(4-chlorophényl)isoxazol-5-yl]méthyl}pipéridin-4-yl)méthylcarbamate de 2-(méthylamino)-2-oxoéthyle ;
- (1-{[5-(4-chlorophényl)-1,3-oxazol-2-yl]méthyl}pipéridin-4-yl)méthylcarbamate de 2-(méthylamino)-2-oxoéthyle ;
- [1-({9-[4-(trifluorométhyl)phényl]-1,3-thiazol-2-yl}méthyl)pipéridin-4-yl]méthylcarbamate de 2-(méthylamino)-2-oxoéthyle ;
- 2-(1-{[3-(4-chlorophényl)isoxazol-5-yl]méthyl}pipéridin-4-yl)éthylcarbamate de 2-(méthylamino)-2-oxoéthyle ;
- 2-(1-{[5-(4-chlorophényl)-1,3-oxazol-2-yl]méthyl}pipéridin-4-yl)éthylcarbamate de 2-(méthylamino)-2-oxoéthyle ;
- 2-[1-({4-[4-(trifluorométhyl)phényl]-1,3-thiazol-2-yl}méthyl)pipéridin-4-yl]éthylcarbamate de 2-(méthylamino)-2-oxoéthyle ;
- (1-{2-[5-(4-chlorophényl)isoxazol-3-yl]éthyl}pipéridin-4-yl)méthylcarbamate de 2-(méthylamino)-2-oxoéthyle ;
- 2-(1-{2-[3-(9-chlorophényl)isoxazol-5-yl]éthyl}pipéridin-4-yl)éthylcarbamate de 2-(méthylamino)-2-oxoéthyle ;
- (1-{2-[3-(4-chlorophényl)isoxazol-5-yl]éthyl}pipéridin-4-yl)méthylcarbamate de 2-(méthylamino)-2-oxoéthyle ;
- (1-{3-[3-(4-chlorophényl)isoxazol-5-yl]propyl}pipéridin-4-yl)méthylcarbamate de 2-(méthylamino)-2-oxoéthyle ;
- (1-{3-[5-(4-chlorophényl)isoxazol-3-yl]propyl}pipéridin-4-yl)méthylcarbamate de 2-(méthylamino)-2-oxoéthyle ;
- {1-[1-(2-chloro-4-fluorophényl)éthyl]pipéridin-4-yl}méthylcarbamate de 2-(méthylamino)-2-oxoéthyle ;
- (1-{1-[3-(4-chlorophénoxy)phényl]éthyl}pipéridin-4-yl)méthylcarbamate de 2-(méthylamino)-2-oxoéthyle ;
- (1-{1-[2-chloro-3-(4-chlorophénoxy)phényl]éthyl}pipéridin-4-yl)méthylcarbamate de 2-(méthylamino)-2-oxoéthyle ;
- 2-(1-{1-[3-(trifluorométhoxy)phényl]éthyl}pipéridin-4-yl)éthylcarbamate de 2-(méthylamino)-2-oxoéthyle ;
- 2-{1-{1-(2-chloro-4-fluorophényl)éthyl]pipéridin-4-yl}éthylcarbamate de 2-(méthylamino)-2-oxoéthyle ;
- {1-[4-(4-chlorophényl)but-3-yn-1-yl]pipéridin-4-yl}carbamate de 2-(méthylamino)-2-oxoéthyle ;
- {1-[5-(4-chlorophényl)pent-4-yn-1-yl]pipéridin-4-yl}carbamate de 2-(méthylamino)-2-oxoéthyle ;
- {1-[5-(2,5-dichlorophényl)pent-4-yn-1-yl]pipéridin-4-yl}carbamate de 2-(méthylamino)-2-oxoéthyle ;
- {1-[4-(4-chlorophényl)but-3-yn-1-yl]pipéridin-4-yl}méthylcarbamate de 2-(méthylamino)-2-oxoéthyle ;
- {1-[4-(4-chloro-2-fluorophényl)but-3-yn-1-yl]pipéridin-4-yl}méthylcarbamate de 2-(méthylamino)-2-oxoéthyle ;
- {1-[4-(2,5-dichlorophényl)but-3-yn-1-yl]pipéridin-4-yl}méthylcarbamate de 2-(méthylamino)-2-oxoéthyle.

8. Composé de formule (I') selon la revendication 1 choisi par les composés suivants :
- {1-[(2-chlorophényl)méthyl]pipéridin-4-yl}méthylcarbamate de 2-(méthylamino)-2-oxoéthyle ;
- {1-[(4-chlorophényl)méthyl]pipéridin-4-yl}méthylcarbamate de 2-(méthylamino)-2-oxoéthyle ;
- {1-[(4-chlorophényl)méthyl]pipéridin-4-yl}carbamate de 2-(méthylamino)-2-oxoéthyle ;
- (1-[4-(1-méthyléthyl)phényl]méthyl pipéridin-4-yl)méthylcarbamate de 2-(méthylamino)-2-oxoéthyle;
- [1-(biphényl-4-ylméthyl)pipéridin-4-yl]carbamate de 2-(méthylamino)-2-oxoéthyle ;
- [1-(biphényl-4-ylméthyl)pipéridin-4-yl]méthylcarbamate de 2-(méthylamino)-2-oxoéthyle ;
- 2-[1-(biphényl-4-ylméthyl)pipéridin-4-yl]éthylcarbamate de 2-(méthylamino)-2-oxoéthyle ;
- [1-(2-biphényl-4-yléthyl)pipéridin-4-yl]carbamate de 2-(méthylamino)-2-oxoéthyle ;
- [1-(naphthalèn-2-ylméthyl)pipéridin-4-yl]méthylcarbamate de 2-(méthylamino)-2-oxoéthyle ;
- 2-{1-[(4-bromophényl)méthyl]pipéridin-4-yl}éthylcarbamate de 2-(méthylamino)-2- oxoéthyle ;
- 2-(1-{[3-(trifluorométhyl)phényl]méthyl}pipéridin-4-yl)éthylcarbamate de 2-(méthylamino)-2-oxoéthyle ;
- 2-(1-{[4-(trifluorométhyl)phényl]méthyl}pipéridin-4-yl)éthylcarbamate de 2-(méthylamino)-2-oxoéthyle ;
- 2-{1-[(2,3-dichlorophényl)méthyl]pipéridin-4-yl}éthylcarbamate de 2-(méthylamino)-2-oxoéthyle ;
- 2-{1-[(3,4-dichlorophényl)méthyl]pipéridin-4-yl}éthylcarbamate de 2-(méthylamino)-2-oxoéthyle ;
- 2-[1-(naphthalèn-1-ylméthyl)pipéridin-4-yl]éthylcarbamate de 2-(méthylamino)-2-oxoéthyle ;
- 2-[1-(naphthalèn-2-ylméthyl)pipéridin-4-yl]éthylcarbamate de 2-(méthylamino)-2-oxoéthyle ;
- 2-[1-(pyridin-2-ylméthyl)pipéridin-4-yl]éthylcarbamate de 2-(méthylamino)-2-oxoéthyle ;
- (1-{2-[4-fluoro-2-(méthyloxy)phényl]éthyl}pipéridin-4-yl)méthylcarbamate de 2-(méthylamino)-2-oxoéthyle ;
- (1-{2-[(4-fluorophényl)oxy]éthyl}pipéridin-4-yl)carbamate de 2-(méthylamino)-2-oxoéthyle ;
- (1-{2-[(4-chlorophényl)oxy]éthyl}pipéridin-4-yl)carbamate de 2-(méthylamino)-2-oxoéthyle ;
- (1-{2-[(2,4-dichlorophényl)oxy]éthyl}pipéridin-4-yl)carbamate de 2-(méthylamino)-2-oxoéthyle ;
- (1-{2-[(4-chlorophényl)oxy]éthyl}pipéridin-4-yl)méthylcarbamate de 2-(méthylamino)-2-oxoéthyle ;
- (1-{2-[(2,4-dichlorophényl)oxy]éthyl} pipéridin-4-yl)méthylcarbamate de 2-(méthylamino)-2-oxoéthyle ;
- 2-(1-{2-[(4-fluorophényl)oxy]éthyl}pipéridin-4-yl)éthylcarbamate de 2-(méthylamino)-2-oxoéthyle ;
- 2-(1-{2-[(4-chlorophényl)oxy]éthyl}pipéridin-4-yl)éthylcarbamate de 2-(méthylamino)-2-oxoéthyle ;
- (1-{2-[(4-fluorophényl)oxy]éthyl} pyrrolidin-3-yl)méthylcarbamate de 2-(méthylamino)-2-oxoéthyle ;
- (1-{2-[(4-chlorophényl)oxy]éthyl}pyrrolidin-3-yl)méthylcarbamate de 2-(méthylamino)-2-oxoéthyle ;
- (1-{2-[3-(trifluorométhyl)phényl]éthyl}pipéridin-4-yl)carbamate de 2-(méthylamino)-2-oxoéthyle ;
- {1-[2-(4-chlorophényl)éthyl]pipéridin-4-yl}carbamate de 2-(méthylamino)-2-oxoéthyle ;
- {1-[2-(4- cyanophényl)éthyl]pipéridin-4-yl}carbamate de 2-(méthylamino)-2-oxoéthyle ;
- (1-{2-[(isoquinolin-5-yl)oxy]éthyl}pipéridin-4-yl)carbamate de 2-(méthylamino)-2-oxoéthyle ;
- [1-(2-naphthalèn-1-yléthyl)pipéridin-4-yl]carbamate de 2-(méthylamino)-2-oxoéthyle ;
- [1-(2-naphthalèn-2-yléthyl)pipéridin-4-yl]carbamate de 2-(méthylamino)-2-oxoéthyle ;
- {1-[3-(4-chlorophényl)propyl]pipéridin-4-yl}carbamate de 2-(méthylamino)-2-oxoéthyle ;
- (1-{3-[4-(méthyloxy)phényl]propyl}pipéridin-4-yl)carbamate de 2-(méthylamino)-2-oxoéthyle ;
- {1-[2-(3-chlorophényl)éthyl]pipéridin-4-yl}méthylcarbamate de 2-(méthylamino)-2-oxoéthyle ;
- (1-{2-[4-(éthyloxy)phényl]éthyl}pipéridin-4-yl)méthylcarbamate de 2-(méthylamino)-2-oxoéthyle ;
- (1-{2-[4-(diméthylamino)phényl]éthyl}pipéridin-4-yl)méthylcarbamate de 2-(méthylamino)-2-oxoéthyle ;
- {1-[2-(2,4-dichlorophényl)éthyl]pipéridin-4-yl}méthylcarbamate de 2-(méthylamino)-2-oxoéthyle ;
- [1-(2-naphthalèn-1-yléthyl)pipéridin-4-yl]méthylcarbamate de 2-(méthylamino)-2-oxoéthyle ;
- [1-(2-naphthalèn-2-yléthyl)pipéridin-4-yl]méthylcarbamate de 2-(méthylamino)-2-oxoéthyle ;
- (1-{3-[4-(méthyloxy)phényl]propyl}pipéridin-4-yl)méthylcarbamate de 2-(méthylamino)-2-oxoéthyle ;
- 2-{1-[2-(2-chlorophényl)éthyl]pipéridin-4-yl}éthylcarbamate de 2-(méthylamino)-2-oxoéthyle ;
- 2-{1-[2-(4-fluorophényl)éthyl]pipéridin-4-yl}éthylcarbamate de 2-(méthylamino)-2-oxoéthyle ;
- 2-(1-{2-[4-(éthyloxy)phényl]éthyl}pipéridin-4-yl)éthylcarbamate de 2-(méthylamino)-2-oxoéthyle ;
- 2-{1-[2-(2-chloro-6-fluorophényl)éthyl]pipéridin-4-yl}éthylcarbamate de 2-(méthylamino)-2-oxoéthyle ;
- 2-(1-{3-[4-(méthyloxy)phényl]propyl}pipéridin-4-yl)éthylcarbamate de 2-(méthylamino)-2-oxoéthyle.

9. Procédé de préparation d'un composé de formule (I) selon l'une quelconque des revendications 1 à 8, comprenant l'étape consistant à
transformer l'oxazolidine-dione de formule générale (IIa) 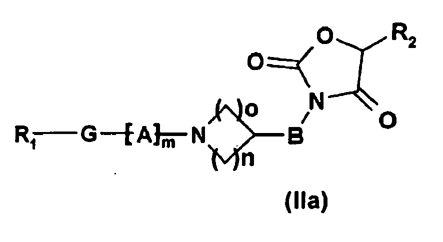 dans laquelle A, B, G, R₁, R₂, m, n et o sont tels que définis dans la formule (I) selon la revendication 1,
par aminolyse au moyen d'une amine de formule générale R₃NH₂ dans laquelle R₃ est tel que défini dans la formule (I) selon la revendication 1.

10. Procédé de préparation d'un composé de formule (I) selon l'une quelconque des revendications 1 à 8, comprenant l'étape consistant à
transformer le dérivé carbamate-amide de formule générale (Ia) 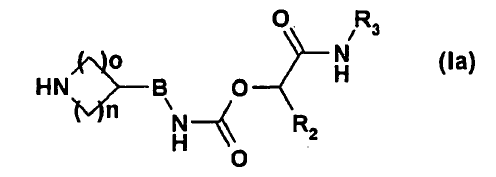 dans laquelle B, R₂, R₃, n et o sont tels que définis dans la formule (I) selon la revendication 1,
par réaction avec un dérivé de formule générale (III) 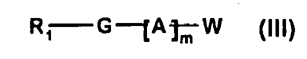 dans laquelle W représente un groupe mésylate, tosylate, ou un atome de chlore, de brome ou d'iode, et m, G, A et R₁ sont tels que définis dans la formule (I) selon la revendication 1.

11. Composé répondant à la formule générale (IIa), 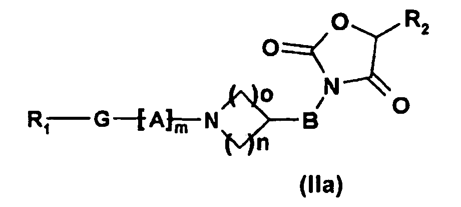 dans laquelle A, B, G, R₁, R₂, m, n et o sont tels que définis dans la formule (I) selon la revendication 1,
le 3-[1-(phénylméthyl)-4-pipéridinyl]-2,4-oxazolidinedione étant exclu.

12. Composé répondant à la formule générale (Ia), 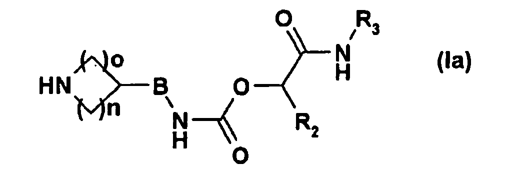 dans laquelle B, R₂, R₃, n et o sont tels que définis dans la formule (I) selon la revendication 1.

13. Composition pharmaceutique contenant au moins un composé de formule (I) selon l'une quelconque des revendications 1 à 8, à l'état de base, de sel, d'hydrate ou de solvat pharmaceutiquement acceptable et éventuellement un ou plusieurs excipients pharmaceutiquement acceptables.

14. Composé de formule (I) selon l'une quelconque des revendications 1 à 8, à l'état de base, de sel, d'hydrate ou de solvat pharmaceutiquement acceptable, pour son utilisation comme médicament.

15. Utilisation d'un composé de formule (I) selon l'une quelconque des revendications 1 à 8, à l'état de base, de sel, d'hydrate ou de solvat pharmaceutiquement acceptable, pour la préparation d'un médicament destiné à prévenir ou à traiter les douleurs aiguës ou chroniques de type neurogène, les douleurs aiguës ou chroniques associées aux maladies inflammatoires, les douleurs aiguës ou chroniques périphériques, les vertiges, les vomissements, les nausées, les troubles du comportement alimentaire, les pathologies neurologiques et psychiatriques, les maladies neuro-dégénératives aiguës ou chroniques, l'épilepsie, les troubles du sommeil, les maladies cardiovasculaires, l'ischémie rénale, les cancers, les désordres du système immunitaire, les maladies allergiques, les maladies infectieuses parasitaires , virales ou bactériennes, les maladies inflammatoires, l'ostéoporose, les affections oculaires, les affections pulmonaires, les maladies gastro-intestinales, l'incontinence urinaire ou l'inflammation vésicale.

## Claims

1. Compound of the formula (I) 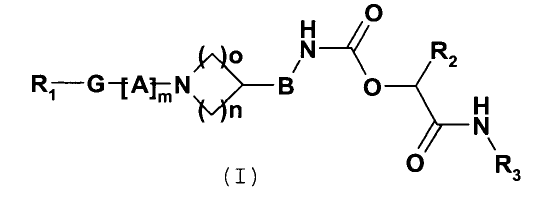 in which
m represents an integer from 1 to 4;
n represents an integer 1, 2 or 3;
o represents an integer 1 or 2;
A is selected from one or more groups X, Y and/or Z;
X represents a methylene group optionally substituted by one or two C₁₋₆ alkyl, C₃₋₇ cycloalkyl or C₃₋₇ cycloalkyl-C₁₋₃ alkylene groups;
Y represents either a C₂ alkenylene group optionally substituted by one or two C₁₋₆ alkyl, C₃₋₇ cycloalkyl or C₃₋₇ cycloalkyl-C₁₋₃ alkylene groups, or a C₂ alkynylene group;
Z represents a group of formula: 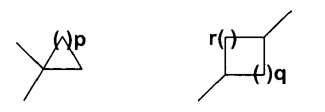
p represents an integer from 1 to 5;
q and r represent integers and are defined such that r+q is a number from 1 to 5;
B represents a covalent bond or a C₁₋₆ alkylene group;
G represents a covalent bond, an oxygen or sulphur atom or a -CH(OH)-, CO, SO or SO₂ group;
R₁ represents a group R₄ optionally substituted by one or more groups R₅ and/or R₆;
R₄ represents a group selected from a furanyl, pyrrolyl, thienyl, oxazolyl, isoxazolyl, thiazolyl, isothiazolyl, imidazolyl, pyrazolyl, thiadiazolyl, isothiadiazolyl, oxadiazolyl, thiadiazolyl, triazolyl, tetrazolyl, phenyl, pyridinyl, pyridazinyl, pyrimidinyl, pyrazinyl, triazinyl, naphthalenyl, quinolinyl, tetrahydroquinolinyl, isoquinolinyl, tetrahydroisoquinolinyl, phthalazinyl, quinazolinyl, quinoxalinyl, cinnolinyl, naphthyridinyl, imidazopyrimidinyl, thienopyrimidinyl, benzofuranyl, dihydrobenzofuranyl, benzothienyl, dihydrobenzothienyl, benzimidazolyl, benzoxazolyl, benzisoxazolyl, benzothiazolyl, benzisothiazolyl, indolyl, isoindolyl, indazolyl, pyrrolopyridinyl, furopyridinyl, dihydrofuropyridinyl, thienopyridinyl, dihydrothienopyridinyl, imidazopyridinyl, pyrazolopyridinyl, oxazolopyridinyl, isoxazolopyridinyl, thiazolopyridinyl;
R₅ represents a halogen atom, a cyano, nitro, hydroxyl, C₁₋₆ alkyl, C₁₋₆ alkoxy, C₁₋₆ thioalkyl, C₁₋₆ fluoroalkyl, C₁₋₆ fluoroalkoxy, C₁₋₆ fluorothioalkyl, C₃₋₇ cycloalkyl or C₃₋₇ cycloalkyl-C₁₋₃ alkylene group or a group NR₇R₈, NR₇COR₈, NR₇CO₂R₈, NR₇SO₂R₈, COR₇, CO₂R₇, CONR₇R₈, SO₂R₇, SO₂NR₇R₈ or -O-(C₁₋₃ alkylene)-O-;
R₆ represents a phenyl, phenyloxy, benzyloxy, pyridinyl, pyrazinyl, pyridazinyl, pyrimidinyl or pyrimidinyloxy group; it being possible for the group or groups R₆ to be substituted by one or more groups R₅ identical to or different from one another;
R₇ and R₈ represent independently of one another a hydrogen atom or a C₁₋₆ alkyl group, or, with the atom or atoms which carry them, form a ring selected from an azetidine, pyrrolidine, piperidine, morpholine, thiomorpholine, azepine or piperazine ring, this ring being optionally substituted by a C₁₋₆ alkyl or benzyl group;
R₂ represents a hydrogen atom or a C₁₋₆ alkyl group;
R₃ represents a hydrogen atom or a C₁₋₆ alkyl, C₃₋₇ cycloalkyl or C₃₋₇ cycloalkyl-C₁₋₃ alkylene group;
in the form of a base, addition salt with an acid, hydrate or solvate.

2. Compound of formula (I) according to Claim 1, **characterized in that:**
m represents an integer from 1 to 4;
n represents an integer 1 or 2;
o represents an integer 1 or 2;
A is selected from one or more groups X and/or Y;
X represents a methylene group optionally substituted by one or two C₁₋₆ alkyl groups;
Y represents a C₂ alkynylene group;
B represents a covalent bond or a C₁₋₆ alkylene group;
G represents a covalent bond or an oxygen atom;
R₁ represents a group R₄ optionally substituted by one or more groups R₅ and/or R₆;
R₄ represents a group selected from an oxazolyl, isoxazolyl, thiazolyl, phenyl, pyridinyl, naphthalenyl, quinolinyl, isoquinolinyl;
R₅ represents a halogen atom, a cyano group, a group NR₇R₈, or a C₁₋₆ alkyl group, a C₁₋₆ alkoxy group, a C₁₋₆ fluoroalkyl group or a C₁₋₆ fluoroalkoxy group;
R₆ represents a phenyl, phenyloxy or pyrimidinyloxy group; it being possible for the group or groups R₆ to be substituted by one or more groups R₅ identical to or different from one another;
R₇ and R₈ represent independently of one another a C₁₋₆ alkyl group;
R₂ represents a hydrogen atom or a C₁₋₆ alkyl group;
R₃ represents a hydrogen atom or a C₁₋₆ alkyl, C₃₋₇ cycloalkyl or C₃₋₇ cycloalkyl-C₁₋₃ alkylene group;
in the form of a base, addition salt with an acid, hydrate or solvate.

3. Compound of formula (I) according to Claim 1 or 2, **characterized in that:**
m represents an integer 1 or 2;
in the form of a base, addition salt with an acid, hydrate or solvate.

4. Compound of formula (I) according to any one of Claims 1 to 3, **characterized in that:**
n is 2;
o is 2;
in the form of a base, addition salt with an acid, hydrate or solvate.

5. Compound of formula (I) according to any one of Claims 1 to 4, **characterized in that:**
R₁ represents a group R₄ optionally substituted by one or more groups R₅ and/or R₆;
R₄ represents a group selected from an oxazolyl, isoxazolyl, phenyl or naphthalenyl;
R₅ represents a halogen atom, a cyano group, a group NR₇R₈, a C₁₋₆ alkyl group, a C₁₋₆ alkoxy group, a C₁₋₆ fluoroalkyl group, or a C₁₋₆ fluoroalkoxy group;
R₆ represents a phenyl, phenyloxy or pyrimidinyloxy group; it being possible for the group or groups R₆ to be substituted by one or more groups R₅ identical to or different from one another;
R₇ and R₈ represent independently of one another a C₁₋₆ alkyl group;
in the form of a base, addition salt with an acid, hydrate or solvate.

6. Compound of formula (I) according to any one of Claims 1 to 5, **characterized in that:**
R₂ represents a hydrogen atom;
R₃ represents a hydrogen atom or a C₁₋₆ alkyl group;
in the form of a base, addition salt with an acid, hydrate or solvate.

7. Compound of formula (I) according to Claim 1 selected from the following compounds:
- 2-(methylamino)-2-oxoethyl {1-[(3,4'-difluorobiphenyl-4-yl)methyl]piperidin-4-yl}methylcarbamate;
- 2-(methylamino)-2-oxoethyl {1-[(3-chloro-4'-fluorobiphenyl-4-yl)methyl]piperidin-4-yl}methylcarbamate;
- 2-(methylamino)-2-oxoethyl {1-[3-(4-fluorophenoxy)benzyl]piperidin-4-yl}methylcarbamate;
- 2-(methylamino)-2-oxoethyl {1-[4-(4-chloro-3-fluorophenoxy)benzyl]piperidin-4-yl}methylcarbamate;
- 2-(methylamino)-2-oxoethyl 2-{1-[3-(trifluoromethoxy)benzyl]piperidin-4-yl}ethylcarbamate;
- 2-(methylamino)-2-oxoethyl 2-{1-[4-(trifluoromethoxy)benzyl]piperidin-4-yl}ethylcarbamate;
- 2-(methylamino)-2-oxoethyl 2-[1-(3-tert-butoxybenzyl)piperidin-4-yl]ethylcarbamate;
- 2-(methylamino)-2-oxoethyl 2-[1-(3-tert-butoxybenzyl)piperidin-4-yl]methylcarbamate;
- 2-(methylamino)-2-oxoethyl 2-[1-(2,4-dichlorobenzyl)piperidin-4-yl]ethylcarbamate;
- 2-(methylamino)-2-oxoethyl 2-[1-(2,5-dichlorobenzyl)piperidin-4-yl]ethylcarbamate;
- 2-(methylamino)-2-oxoethyl 2-[1-(3,5-dichlorobenzyl)piperidin-4-yl]ethylcarbamate;
- 2-(methylamino)-2-oxoethyl 2-[1-(2-chloro-5-fluorobenzyl)piperidin-4-yl]ethylcarbamate;
- 2-(methylamino)-2-oxoethyl 2-[1-(3-chloro-2-fluorobenzyl)piperidin-4-yl]ethylcarbamate;
- 2-(methylamino)-2-oxoethyl 2-[1-(3-chloro-5-methylbenzyl)piperidin-4-yl]ethylcarbamate;
- 2-(methylamino)-2-oxoethyl 2-{1-[(3,4'-difluorobiphenyl-4-yl)methyl]piperidin-4-yl}ethylcarbamate;
- 2-(methylamino)-2-oxoethyl 2-{1-[(3-chloro-4'-fluorobiphenyl-4-yl)methyl]piperidin-4-yl}ethylcarbamate;
- 2-(methylamino)-2-oxoethyl 2-{1-[4-(4-chloro-3-fluorophenoxy)benzyl]piperidin-4-yl}ethylcarbamate;
- 2-(methylamino)-2-oxoethyl {1-[3-(4-fluorophenoxy)benzyl]piperidin-4-yl}carbamate;
- 2-(methylamino)-2-oxoethyl {1-[3-(trifluoromethoxy)benzyl]piperidin-4-yl}methylcarbamate;
- 2-(methylamino)-2-oxoethyl {1-[4-(trifluoromethoxy)benzyl]piperidin-4-yl}methylcarbamate;
- 2-(methylamino)-2-oxoethyl {1-[3-(pyrimidin-2-yloxy)benzyl]piperidin-4-yl}methylcarbamate;
- 2-(methylamino)-2-oxoethyl 2-[1-(2-chloro-4-fluorobenzyl)piperidin-4-yl]ethylcarbamate;
- 2-(methylamino)-2-oxoethyl 2-[1-(3-chloro-4-fluorobenzyl)piperidin-4-yl]ethylcarbamate;
- 2-(methylamino)-2-oxoethyl 2-[1-(3-cyano-5-fluorobenzyl)piperidin-4-yl]ethylcarbamate;
- 2-(methylamino)-2-oxoethyl 2-{1-[3-(4-fluorophenoxy)benzyl]piperidin-4-yl}ethylcarbamate;
- 2-(methylamino)-2-oxoethyl (1-{[3-(4-chlorophenyl)isoxazol-5-yl]methyl}piperidin-4-yl)methylcarbamate;
- 2-(methylamino)-2-oxoethyl (1-{[5-(4-chlorophenyl)-1,3-oxazol-2-yl]methyl}piperidin-4-yl)methylcarbamate;
- 2-(methylamino)-2-oxoethyl [1-({4-[4-(trifluoromethyl)phenyl]-1,3-thiazol-2-yl}methyl)piperidin-4-yl]methylcarbamate;
- 2-(methylamino)-2-oxoethyl 2-(1-{[3-(4-chlorophenyl)isoxazol-5-yl]methyl}piperidin-4-yl)ethylcarbamate;
- 2-(methylamino)-2-oxoethyl 2-(1-{[5-(4-chlorophenyl)-1,3-oxazol-2-yl]methyl}piperidin-4-yl)ethylcarbamate;
- 2-(methylamino)-2-oxoethyl 2-[1-({4-[4-(trifluoromethyl)phenyl]-1,3-thiazol-2-yl}methyl)piperidin-4-yl]ethylcarbamate;
- 2-(methylamino)-2-oxoethyl (1-{2-[5-(4-chlorophenyl)isoxazol-3-yl]ethyl}piperidin-4-yl)methylcarbamate;
- 2-(methylamino)-2-oxoethyl 2-(1-{2-[3-(4-chlorophenyl)isoxazol-5-yl]ethyl}piperidin-4-yl)ethylcarbamate;
- 2-(methylamino)-2-oxoethyl (1-{2-[3-(4-chlorophenyl)isoxazol-5-yl]ethyl}piperidin-4-yl)methylcarbamate;
- 2-(methylamino)-2-oxoethyl (1-{3-[3-(4-chlorophenyl)isoxazol-5-yl]propyl}piperidin-4-yl)methylcarbamate;
- 2-(methylamino)-2-oxoethyl (1-{3-[5-(4-chlorophenyl)isoxazol-3-yl]propyl}piperidin-4-yl)methylcarbamate;
- 2-(methylamino)-2-oxoethyl {1-[1-(2-chloro-4-fluorophenyl)ethyl]piperidin-4-yl}methylcarbamate;
- 2-(methylamino)-2-oxoethyl (1-{1-[3-(4-chlorophenoxy)phenyl]ethyl}piperidin-4-yl)methylcarbamate;
- 2-(methylamino)-2-oxoethyl (1-{1-[2-chloro-3-(4-chlorophenoxy)phenyl]ethyl}piperidin-4-yl)methylcarbamate;
- 2-(methylamino)-2-oxoethyl 2-(1-{1-[3-(trifluoromethoxy)phenyl]ethyl}piperidin-4-yl)ethylcarbamate;
- 2-(methylamino)-2-oxoethyl 2-{1-[1-(2-chloro-4-fluorophenyl)ethyl]piperidin-4-yl}ethylcarbamate;
- 2-(methylamino)-2-oxoethyl {1-[4-(4-chlorophenyl)but-3-yn-1-yl]piperidin-4-yl}carbamate;
- 2-(methylamino)-2-oxoethyl {1-[5-(4-chlorophenyl)pent-4-yn-1-yl]piperidin-4-yl}carbamate;
- 2-(methylamino)-2-oxoethyl {1-[5-(2,5-dichlorophenyl)pent-4-yn-1-yl]piperidin-4-yl}carbamate;
- 2-(methylamino)-2-oxoethyl {1-[4-(4-chlorophenyl)but-3-yn-1-yl]piperidin-4-yl}methylcarbamate;
- 2-(methylamino)-2-oxoethyl {1-[4-(4-chloro-2-fluorophenyl)but-3-yn-1-yl]piperidin-4-yl}methylcarbamate;
- 2-(methylamino)-2-oxoethyl {1-[4-(2,5-dichlorophenyl)but-3-yn-1-yl]piperidin-4-yl}methylcarbamate.

8. Compound of formula (I') according to Claim 1 selected from the following compounds:
- 2-(methylamino)-2-oxoethyl {1-[(2-chlorophenyl)-methyl]piperidin-4-yl}methylcarbamate;
- 2-(methylamino)-2-oxoethyl {1-[(4-chlorophenyl)-methyl]piperidin-4-yl}methylcarbamate;
- 2-(methylamino)-2-oxoethyl {1-[(4-chlorophenyl)-methyl]piperidin-4-yl}carbamate;
- 2-(methylamino)-2-oxoethyl (1-[4-(1-methylethyl)-phenyl]methylpiperidin-4-yl)methylcarbamate;
- 2-(methylamino)-2-oxoethyl [1-(biphenyl-4-ylmethyl)piperidin-4-yl]carbamate;
- 2-(methylamino)-2-oxoethyl [1-(biphenyl-4-ylmethyl)piperidin-4-yl]methylcarbamate;
- 2-(methylamino)-2-oxoethyl 2-[1-(biphenyl-4-yl-methyl)piperidin-4-yl]ethylcarbamate;
- 2-(methylamino)-2-oxoethyl [1-(2-biphenyl-4-ylethyl)piperidin-4-yl]carbamate;
- 2-(methylamino)-2-oxoethyl [1-(naphthalen-2-yl-methyl)piperidin-4-yl]methylcarbamate;
- 2-(methylamino)-2-oxoethyl 2-{1-[(4-bromophenyl)-methyl]piperidin-4-yl}ethylcarbamate;
- 2-(methylamino)-2-oxoethyl 2-(1-{[3-(trifluoromethyl)phenyl]methyl}piperidin-4-yl)ethylcarbamate;
- 2-(methylamino)-2-oxoethyl 2-(1-{[4-(trifluoromethyl)phenyl]methyl}piperidin-4-yl)ethylcarbamate;
- 2-(methylamino)-2-oxoethyl 2-{1-[(2,3-dichlorophenyl)methyl]piperidin-4-yl}ethylcarbamate;
- 2-(methylamino)-2-oxoethyl 2-{1-[(3,4-dichlorophenyl)methyl]piperidin-4-yl}ethylcarbamate;
- 2-(methylamino)-2-oxoethyl 2-[1-(naphthalen-1-yl-methyl)piperidin-4-yl]ethylcarbamate;
- 2-(methylamino)-2-oxoethyl 2-[1-(naphthalen-2-yl-methyl)piperidin-4-yl]ethylcarbamate;
- 2-(methylamino)-2-oxoethyl 2-[1-(pyridin-2-yl-methyl)piperidin-4-yl]ethylcarbamate;
- 2-(methylamino)-2-oxoethyl (1-{2-[4-fluoro-2-(methyloxy)phenyl]ethyl}piperidin-4-yl)methylcarbamate;
- 2-(methylamino)-2-oxoethyl (1-{2-[(4-fluorophenyl)oxy]ethyl}piperidin-4-yl)carbamate;
- 2-(methylamino)-2-oxoethyl (1-{2-[(4-chlorophenyl)oxy]ethyl}piperidin-4-yl)carbamate;
- 2-(methylamino)-2-oxoethyl (1-{2-[(2,4-dichlorophenyl)oxy]ethyl}piperidin-4-yl)carbamate;
- 2-(methylamino)-2-oxoethyl (1-{2-[(4-chlorophenyl)oxy]ethyl}piperidin-4-yl)methylcarbamate;
- 2-(methylamino)-2-oxoethyl 1-{2-[(2,4-dichlorophenyl)oxy]ethyl}piperidin-4-yl)methylcarbamate;
- 2-(methylamino)-2-oxoethyl 2-(1-{2-[(4-fluorophenyl)oxy]ethyl}piperidin-4-yl)ethylcarbamate;
- 2-(methylamino)-2-oxoethyl 2-(1-{2-[(4-chlorophenyl)oxy]ethyl}piperidin-4-yl)ethylcarbamate;
- 2-(methylamino)-2-oxoethyl (1-{2-[(4-fluorophenyl)oxy]ethyl}pyrrolidin-3-yl)methylcarbamate;
- 2-(methylamino)-2-oxoethyl (1-{2-[(4-chlorophenyl)oxy]ethyl}pyrrolidin-3-yl)methylcarbamate;
- 2-(methylamino)-2-oxoethyl (1-{2-[3-(trifluoromethyl)phenyl]ethyl}piperidin-4-yl)carbamate;
- 2-(methylamino)-2-oxoethyl {1-[2-(4-chlorophenyl)ethyl]piperidin-4-yl}carbamate;
- 2-(methylamino)-2-oxoethyl {1-[2-(4-cyanophenyl)-ethyl]piperidin-4-yl}carbamate;
- 2-(methylamino)-2-oxoethyl (1-{2-[(isoquinolin-5-yl)oxy]ethyl}piperidin-4-yl)carbamate;
- 2-(methylamino)-2-oxoethyl [1-(2-naphthalen-1-yl-ethyl)piperidin-4-yl]carbamate;
- 2-(methylamino)-2-oxoethyl [1-(2-naphthalen-2-yl-ethyl)piperidin-4-yl]carbamate;
- 2-(methylamino)-2-oxoethyl {1-[3-(4-chlorophenyl)propyl]piperidin-4-yl}carbamate;
- 2-(methylamino)-2-oxoethyl (1-{3-[4-(methyloxy)-phenyl]propyl}piperidin-4-yl)carbamate;
- 2-(methylamino)-2-oxoethyl {1-[2-(3-chlorophenyl)ethyl]piperidin-4-yl}methylcarbamate;
- 2-(methylamino)-2-oxoethyl (1-{2-[4-(ethyloxy)-phenyl]ethyl}piperidin-4-yl)methylcarbamate;
- 2-(methylamino)-2-oxoethyl (1-{2-[4-(dimethylamino)phenyl]ethyl}piperidin-4-yl)methylcarbamate;
- 2-(methylamino)-2-oxoethyl {1-[2-(2,4-dichlorophenyl)ethyl]piperidin-4-yl}methylcarbamate;
- 2-(methylamino)-2-oxoethyl [1-(2-naphthalen-1-yl-ethyl)piperidin-4-yl]methylcarbamate;
- 2-(methylamino)-2-oxoethyl [1-(2-naphthalen-2-yl-ethyl)piperidin-4-yl]methylcarbamate;
- 2-(methylamino)-2-oxoethyl (1-{3-[4-(methyloxy)-phenyl]propyl}piperidin-4-yl)methylcarbamate;
- 2-(methylamino)-2-oxoethyl 2-{1-[2-(2-chlorophenyl)ethyl]piperidin-4-yl}ethylcarbamate;
- 2-(methylamino)-2-oxoethyl 2-{1-[2-(4-fluorophenyl)ethyl]piperidin-4-yl}ethylcarbamate;
- 2-(methylamino)-2-oxoethyl 2-(1-{2-[4-(ethyloxy)-phenyl]ethyl}piperidin-4-yl)ethylcarbamate;
- 2-(methylamino)-2-oxoethyl 2-{1-[2-(2-chloro-6-fluorophenyl)ethyl]piperidin-4-yl}ethylcarbamate;
- 2-(methylamino)-2-oxoethyl 2-(1-{3-[4-(methyloxy)phenyl]propyl}piperidin-4-yl)ethylcarbamate.

9. Process for preparing a compound of formula (I) according to any one of Claims 1 to 8, comprising the step consisting in converting the oxazolidine-dione of general formula (IIa) 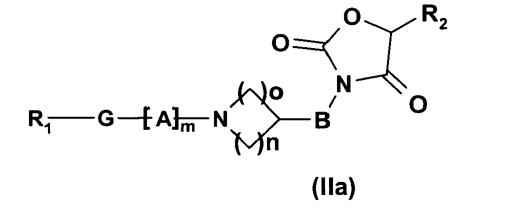 in which A, B, G, R₁, R₂, m, n and o are as defined in the formula (I) according to Claim 1 by aminolysis using an amine of general formula R₃NH₂ in which R₃ is as defined in the formula (I) according to Claim 1.

10. Process for preparing a compound of formula (I) according to any one of Claims 1 to 8, comprising the step consisting in converting the carbamate-amide derivative of general formula (Ia) 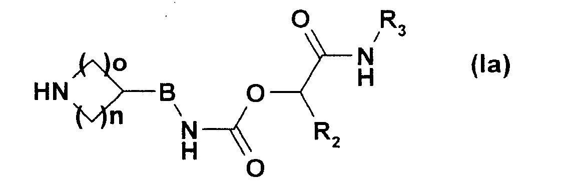 in which B, R₂, R₃, n and o are as defined in the formula (I) according to Claim 1, by reaction with a derivative of general formula (III) 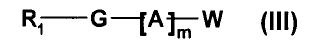 in which W represents a mesylate or tosylate group or a chlorine, bromine or iodine atom and m, G, A and R₁ are as defined in the formula (I) according to Claim 1.

11. Compound of the general formula (IIa) 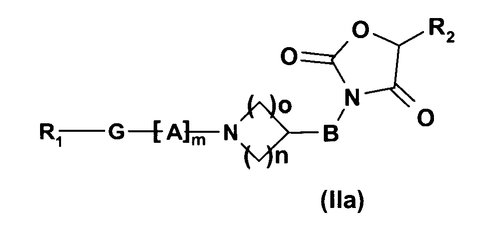 in which A, B, G, R₁, R₂, m, n and o are as defined in the formula (I) according to Claim 1,
with the exception of 3-[1-(phenylmethyl)-4-piperidinyl]-2,4-oxazolidinedione.

12. Compound of the general formula (Ia) 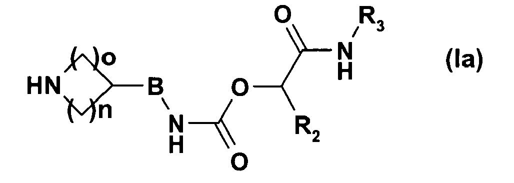 in which B, R₂, R₃, n and o are as defined in the formula (I) according to Claim 1.

13. Pharmaceutical composition comprising at least one compound of formula (I) according to any one of Claims 1 to 8, in base, salt, hydrate or pharmaceutically acceptable solvate form, and, optionally, one or more pharmaceutically acceptable excipients.

14. Compound of formula (I) according to any one of Claims 1 to 8, in base, salt, hydrate or pharmaceutically acceptable solvate form, for its use as a medicinal product.

15. Use of a compound of formula (I) according to any one of Claims 1 to 8, in base, salt, hydrate or pharmaceutically acceptable solvate form, for preparing a medicinal product intended for preventing or treating acute or chronic pain of neurogenic type, acute or chronic pain associated with inflammatory diseases, acute or chronic peripheral pain, dizziness, vomiting, nausea, eating disorders, neurological and psychiatric pathologies, acute or chronic neurodegenerative diseases, epilepsy, sleep disorders, cardiovascular diseases, renal ischemia, cancers, disorders of the immune system, allergic diseases, parasitic, viral or bacterial infectious diseases, inflammatory diseases, osteoporosis, ocular conditions, pulmonary conditions, gastrointestinal diseases, urinary incontinence or bladder inflammation.

## Patentansprüche

1. Verbindung der Formel (I) 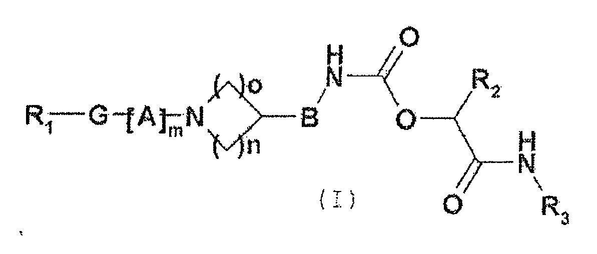 worin
m für eine ganze Zahl von 1 bis 4 steht;
n für eine ganze Zahl mit einem Wert von 1, 2 oder 3 steht;
o für eine ganze Zahl mit einem Wert von 1 oder 2 steht;
A unter einer oder mehreren Gruppen X, Y und/oder Z ausgewählt ist;
X für eine gegebenenfalls durch eine oder zwei C₁₋₆-Alkyl-, C₃₋₇-Cycloalkyl- oder C₃₋₇-Cycloalkyl-C₁₋₃-alkylengruppen substituierte Methylengruppe steht;
Y entweder für eine gegebenenfalls durch eine oder zwei C₁₋₆-Alkyl-, C₃₋₇-Cycloalkyl- oder C₃₋₇-Cycloalkyl-C₁₋₃-alkylengruppen substituierte C₂-Alkenylengruppe oder eine C₂-Alkinylengruppe steht;
Z für eine Gruppe der Formel: 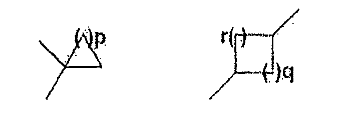 steht;
p für eine ganze Zahl von 1 bis 5 steht;
q und r für ganze Zahlen stehen und so definiert sind, daß r+q eine Zahl von 1 bis 5 ist;
B für eine kovalente Bindung oder eine C₁₋₆-Alkylengruppe steht;
G für eine kovalente Bindung, ein Sauerstoff- oder Schwefelatom oder eine CH(OH)-, CO-, SO- oder SO₂-Gruppe steht;
R₁ für eine gegebenenfalls durch eine oder mehrere Gruppen R₅ und/oder R₆ substituierte Gruppe R₄ steht;
R₄ für eine unter Furanyl, Pyrrolyl, Thienyl, Oxazolyl, Isoxazolyl, Thiazolyl, Isothiazolyl, Imidazolyl, Pyrazolyl, Thiadiazolyl, Isothiadiazolyl, Oxadiazolyl, Thiadiazolyl, Triazolyl, Tetrazolyl, Phenyl, Pyridinyl, Pyridazinyl, Pyrimidinyl, Pyrazinyl, Triazinyl, Naphthalinyl, Chinolinyl, Tetrahydrochinolinyl, Isochinolinyl, Tetrahydroisochinolinyl, Phthalazinyl, Chinazolinyl, Chinoxalinyl, Cinnolinyl, Naphthyridinyl, Imidazopyridinyl, Thienopyrimidinyl, Benzofuranyl, Dihydrobenzofuranyl, Benzothienyl, Dihydrobenzothienyl, Benzimidazolyl, Benzoxazolyl, Benzisoxazolyl, Benzothiazolyl, Benzisothiazolyl, Indolyl, Isoindolyl, Indazolyl, Pyrrolopyridinyl, Furopyridinyl, Dihydrofuropyridinyl, Thienopyridinyl, Dihydrothienopyridinyl, Imidazopyridinyl, Pyrazolopyridinyl, Oxazolopyridinyl, Isoxazolopyridinyl und Thiazolopyridinyl ausgewählte Gruppe steht;
R₅ für ein Halogenatom oder eine Cyano-, Nitro-, Hydroxyl-, C₁₋₆-Alkyl-, C₁₋₆-Alkoxy-, C₁₋₆-Thioalkyl-, C₁₋₆-Fluoralkyl-, C₁₋₆-Fluoralkoxy-, C₁-C₆-Fluorthioalkyl-, C₃₋₇-Cycloalkyl-, C₃₋₇-Cycloalkyl-C₁₋₃-alkylen-, NR₇R₈-, NR₇COR₈-, NR₇CO₂R₈-, NR₇SO₂R₈-, COR₇-, CO₂R₇-, CONR₇R₈-, SO₂R₇-, SO₂NR₇R₈- oder -O-(C₁₋₃-Alkylen)-O-Gruppe steht;
R₆ für eine Phenyl-, Phenyloxy-, Benzyloxy-, Pyridinyl-, Pyrazinyl-, Pyridazinyl-, Pyrimidinyl- oder Pyrimidinyloxygruppe steht; wobei die Gruppe bzw. Gruppen R₆ gegebenenfalls durch eine oder mehrere gleiche oder voneinander verschiedene Gruppen R₅ substituiert sein kann bzw. können;
R₇ und R₈ unabhängig voneinander für ein Wasserstoffatom oder eine C₁₋₆-Alkylgruppe stehen oder mit dem Atom bzw. den Atomen, an das bzw. die sie gebunden sind, einen unter Azetidin, Pyrrolidin, Piperidin, Morpholin, Thiomorpholin, Azepin und Piperazin ausgewählten Ring bilden, welcher gegebenenfalls durch eine C₁₋₆-Alkyl- oder Benzylgruppe substituiert ist;
R₂ für ein Wasserstoffatom oder eine C₁₋₆-Alkylgruppe steht;
R₃ für ein Wasserstoffatom oder eine C₁₋₆-Alkyl-, C₃₋₇-Cycloalkyl- oder C₃₋₇-Cycloalkyl-C₁₋₃-alkylengruppe steht;
in Basen-, Säureadditionssalz-, Hydrat- oder Solvatform.

2. Verbindung der Formel (I) nach Anspruch 1, **dadurch gekennzeichnet, daß**
m für eine ganze Zahl von 1 bis 4 steht;
n für eine ganze Zahl mit einem Wert von 1 oder 2 steht;
o für eine ganze Zahl mit einem Wert von 1 oder 2 steht;
A unter einer oder mehreren Gruppen X und/oder Y ausgewählt ist;
X für eine gegebenenfalls durch eine oder zwei C₁₋₆-Alkylgruppen substituierte Methylengruppe steht;
Y für eine C₂-Alkinylengruppe steht;
B für eine kovalente Bindung oder eine C₁₋₆-Alkylengruppe steht;
G für eine kovalente Bindung oder ein Sauerstoffatom steht;
R₁ für eine gegebenenfalls durch eine oder mehrere Gruppen R₅ und/oder R₆ substituierte Gruppe R₄ steht;
R₄ für eine unter Oxazolyl, Isoxazolyl, Thiazolyl, Phenyl, Pyridinyl, Napthalinyl, Chinolinyl und Isochinolinyl ausgewählte Gruppe steht;
R₅ für ein Halogenatom oder eine Cyano-, NR₇R₈-, C₁₋₆-Alkyl-, C₁₋₆-Alkoxy-, C₁₋₆-Fluoralkyl- oder C₁₋₆-Fluoralkoxygruppe steht;
R₆ für eine Phenyl-, Phenyloxy- oder Pyrimidinyloxygruppe steht; wobei die Gruppe bzw. Gruppen R₆ gegebenenfalls durch eine oder mehrere gleiche oder voneinander verschiedene Gruppen R₅ substituiert sein kann bzw. können;
R₇ und R₈ unabhängig voneinander für eine C₁₋₆-Alkylgruppe stehen
R₂ für ein Wasserstoffatom oder eine C₁₋₆-Alkylgruppe steht;
R₃ für ein Wasserstoffatom oder eine C₁₋₆-Alkyl-, C₃₋₇-Cycloalkyl- oder C₃₋₇-Cycloalkyl-C₁₋₃-alkylengruppe steht;
in Basen-, Säureadditionssalz-, Hydrat- oder Solvatform.

3. Verbindung der Formel (I) nach Anspruch 1 oder 2, **dadurch gekennzeichnet, daß**
m für eine ganze Zahl mit einem Wert von 1 oder 2 steht;
in Basen-, Säureadditionssalz-, Hydrat- oder Solvatform.

4. Verbindung der Formel (I) nach einem der Ansprüche 1 bis 3, **dadurch gekennzeichnet, daß**
n gleich 2 ist;
o gleich 2 ist;
in Basen-, Säureadditionssalz-, Hydrat- oder Solvatform.

5. Verbindung der Formel (I) nach einem der Ansprüche 1 bis 4, **dadurch gekennzeichnet, daß**
R₁ für eine gegebenenfalls durch eine oder mehrere Gruppen R₅ und/oder R₆ substituierte Gruppe R₄ steht;
R₄ für eine unter Oxazolyl, Isoxazolyl, Phenyl oder Naphthalinyl ausgewählte Gruppe steht;
R₅ für ein Halogenatom oder eine Cyano-, NR₇R₈-, C₁₋₆-Alkyl-, C₁₋₆-Alkoxy-, C₁₋₆-Fluoralkyl- oder C₁₋₆-Fluoralkoxygruppe steht;
R₆ für eine Phenyl-, Phenyloxy- oder Pyrimidinyloxygruppe steht; wobei die Gruppe bzw. Gruppen R₆ gegebenenfalls durch eine oder mehrere gleiche oder voneinander verschiedene Gruppen R₅ substituiert sein kann bzw. können;
R₇ und R₈ unabhängig voneinander für eine C₁₋₆-Alkylgruppe stehen;
in Basen-, Säureadditionssalz-, Hydrat- oder Solvatform.

6. Verbindung der Formel (I) nach einem der Ansprüche 1 bis 5, **dadurch gekennzeichnet, daß**
R₂ für ein Wasserstoffatom steht;
R₃ für ein Wasserstoffatom oder eine C₁₋₆-Alkylgruppe steht;
in Basen-, Säureadditionssalz-, Hydrat- oder Solvatform.

7. Verbindung der Formel (I) nach Anspruch 1, ausgewählt unter den folgenden Verbindungen:
- {1-[(3,4'-Difluorbiphenyl-4-yl)methyl]piperidin-4-yl}methylcarbamidsäure-2-(methylamino)-2-oxoethylester;
- {1-[(3-Chlor-4'-fluorbiphenyl-4-yl)methyl]-piperidin-4-yl}methylcarbamidsäure-2-(methylamino)-2-oxoethylester;
- {1-[3-(4-Fluorphenoxy)benzyl]piperidin-4-yl}methylcarbamidsäure-2-(methylamino)-2-oxoethylester;
- {1-[4-(4-Chlor-3-fluorphenoxy)benzyl]piperidin-4-yl}methylcarbamidsäure-2-(methylamino)-2-oxoethylester;
- 2-{1-[3-(Trifluormethoxy)benzyl]piperidin-4-yl}ethylcarbamidsäure-2-(methylamino)-2-oxoethylester;
- 2-{1-[4-(Trifluormethoxy)benzyl]piperidin-4-yl}ethylcarbamidsäure-2-(methylamino)-2-oxoethylester;
- 2-[1-(3-tert.-Butoxybenzyl)piperidin-4-yl]ethylcarbamidsäure-2-(methylamino)-2-oxoethylester;
- 2-[1-(3-tert.-Butoxybenzyl)piperidin-4-yl]-methylcarbamidsäure-2-(methylamino)-2-oxoethylester;
- 2-[1-(2,4-Dichlorbenzyl)piperidin-4-yl]ethylcarbamidsäure-2-(methylamino)-2-oxoethylester;
- 2-[1-(2,5-Dichlorbenzyl)piperidin-4-yl]ethylcarbamidsäure-2-(methylamino)-2-oxoethylester;
- 2-[1-(3,5-Dichlorbenzyl)piperidin-4-yl]ethylcarbamidsäure-2-(methylamino)-2-oxoethylester;
- 2-[1-(2-Chlor-5-fluorbenzyl)piperidin-4-yl]-ethylcarbamidsäure-2-(methylamino)-2-oxoethylester;
- 2-[1-(3-Chlor-2-fluorbenzyl)piperidin-4-yl]-ethylcarbamidsäure-2-(methylamino)-2-oxoethylester;
- 2-[1-(3-Chlor-5-methylbenzyl)piperidin-4-yl]-ethylcarbamidsäure-2-(methylamino)-2-oxoethylester;
- 2-{1-[(3,4'-Difluorbiphenyl-4-yl)methyl]-piperidin-4-yl}ethylcarbamidsäure-2-(methylamino)-2-oxoethylester;
- 2-{1-[(3-Chlor-4'-fluorbiphenyl-4-yl)methyl]-piperidin-4-yl}ethylcarbamidsäure-2-(methylamino)-2-oxoethylester;
- 2-{1-[4-(4-Chlor-3-fluorphenoxy)benzyl]-piperidin-4-yl}ethylcarbamidsäure-2-(methylamino)-2-oxoethylester;
- {1-[3-(4-Fluorphenoxy)benzyl]piperidin-4-yl}carbamidsäure-2-(methylamino)-2-oxoethylester;
- {1-[3-(Trifluormethoxy)benzyl]piperidin-4-yl}methylcarbamidsäure-2-(methylamino)-2-oxoethylester;
- {1-[4-(Trifluormethoxy)benzyl]piperidin-4-yl}methylcarbamidsäure-2-(methylamino)-2-oxoethylester;
- {1-[3-(Pyrimidin-2-yloxy)benzyl]piperidin-4-yl}methylcarbamidsäure-2-(methylamino)-2-oxoethylester;
- 2-[1-(2-Chlor-4-fluorbenzyl)piperidin-4-yl]-ethylcarbamidsäure-2-(methylamino)-2-oxoethylester;
- 2-[1-(3-Chlor-4-fluorbenzyl)piperidin-4-yl]-ethylcarbamidsäure-2-(methylamino)-2-oxoethylester;
- 2-[1-(3-Cyano-5-fluorbenzyl)piperidin-4-yl]-ethylcarbamidsäure-2-(methylamino)-2-oxoethylester;
- 2-{1-[3-(4-Fluorphenoxy)benzyl]piperidin-4-yl}ethylcarbamidsäure-2-(methylamino)-2-oxoethylester;
- (1-{[3-(4-Chlorphenyl)isoxazol-5-yl]methyl}piperidin-4-yl)methylcarbamidsäure-2-(methylamino)-2-oxoethylester;
- (1-{[5-(4-Chlorphenyl)-1,3-oxazol-2-yl]methyl}piperidin-4-yl)methylcarbamidsäure-2-(methylamino)-2-oxoethylester;
- [1-({4-[4-(trifluormethyl)phenyl]-1,3-thiazol-2-yl}methyl)piperidin-4-yl]methylcarbamidsäure-2-(methylamino)-2-oxoethylester;
- 2-(1-{[3-(4-Chlorphenyl)isoxazol-5-yl]methyl}piperidin-4-yl)ethylcarbamidsäure-2-(methylamino)-2-oxoethylester;
- 2-(1-{[5-(4-Chlorphenyl)-1,3-oxazol-2-yl]-methyl}piperidin-4-yl)ethylcarbamidsäure-2-(methylamino)-2-oxoethylester;
- 2-[1-({4-[4-(trifluormethyl)phenyl]-1,3-thiazol-2-yl}methyl)piperidin-4-yl]ethylcarbamidsäure-2-(methylamino)-2-oxoethylester;
- (1-{2-[5-(4-Chlorphenyl)isoxazol-3-yl]ethyl}piperidin-4-yl)methylcarbamidsäure-2-(methylamino)-2-oxoethylester;
- 2-(1-{2-[3-(4-Chlorphenyl)isoxazol-5-yl]ethyl}piperidin-4-yl)ethylcarbamidsäure-2-(methylamino)-2-oxoethylester;
- (1-{2-[3-(4-Chlorphenyl)isoxazol-5-yl]ethyl}piperidin-4-yl)methylcarbamidsäure-2-(methylamino)-2-oxoethylester;
- (1-{3-[3-(4-Chlorphenyl)isoxazol-5-yl]propyl}piperidin-4-yl)methylcarbamidsäure-2-(methylamino)-2-oxoethylester;
- (1-{3-[5-(4-Chlorphenyl)isoxazol-3-yl]propyl}piperidin-4-yl)methylcarbamidsäure-2-(methylamino)-2-oxoethylester;
- {1-[1-(2-Chlor-4-fluorphenyl)ethyl]piperidin-4-yl}methylcarbamidsäure-2-(methylamino)-2-oxoethylester;
- (1-{1-[3-(4-Chlorphenoxy)phenyl]ethyl}piperidin-4-yl)methylcarbamidsäure-2-(methylamino)-2-oxoethylester;
- (1-{1-[2-Chlor-3-(4-chlorphenoxy)phenyl]ethyl}piperidin-4-yl)methylcarbamidsäure-2-(methylamino)-2-oxoethylester;
- 2-(1-{1-[3-(Trifluormethoxy)phenyl]ethyl}piperidin-4-yl)ethylcarbamidsäure-2-(methylamino)-2-oxoethylester;
- 2-{1-[1-(2-Chlor-4-fluorphenyl)ethyl]piperidin-4-yl}ethylcarbamidsäure-2-(methylamino)-2-oxoethylester;
- {1-[4-(4-Chlorphenyl)but-3-in-1-yl]piperidin-4-yl}carbamidsäure-2-(methylamino)-2-oxoethylester;
- {1-[5-(4-Chlorphenyl)pent-4-in-1-yl]piperidin-4-yl}carbamidsäure-2-(methylamino)-2-oxoethylester;
- {1-[5-(2,5-Dichlorphenyl)pent-4-in-1-yl]-piperidin-4-yl}carbamidsäure-2-(methylamino)-2-oxoethylester;
- {1-[4-(4-Chlorphenyl)but-3-in-1-yl]piperidin-4-yl}methylcarbamidsäure-2-(methylamino)-2-oxoethylester;
- {1-[4-(4-Chlor-2-fluorphenyl)but-3-in-1-yl]-piperidin-4-yl}methylcarbamidsäure-2-(methylamino)-2-oxoethylester;
- {1-[4-(2,5-Dichlorphenyl)but-3-in-1-yl]-piperidin-4-yl}methylcarbamidsäure-2-(methylamino)-2-oxoethylester.

8. Verbindung der Formel (I') nach Anspruch 1, ausgewählt unter den folgenden Verbindungen:
- {1-[(2-Chlorphenyl)methyl]piperidin-4-yl}methylcarbamidsäure-2-(methylamino)-2-oxoethylester;
- {1-[(4-Chlorphenyl)methyl]piperidin-4-yl}methylcarbamidsäure-2-(methylamino)-2-oxoethylester;
- {1-[(4-Chlorphenyl)methyl]piperidin-4-yl}carbamidsäure-2-(methylamino)-2-oxoethylester;
- (1-[4-(1-Methylethyl)phenyl]methylpiperidin-4-yl)methylcarbamidsäure-2-(methylamino)-2-oxoethylester;
- [1-(Biphenyl-4-ylmethyl)piperidin-4-yl]-carbamidsäure-2-(methylamino)-2-oxoethylester;
- [1-(Biphenyl-4-ylmethyl)piperidin-4-yl]methylcarbamidsäure-2-(methylamino)-2-oxoethylester;
- 2-[1-(Biphenyl-4-ylmethyl)piperidin-4-yl]ethylcarbamidsäure-2-(methylamino)-2-oxoethylester;
- [1-(2-Biphenyl-4-ylethyl)piperidin-4-yl]-carbamidsäure-2-(methylamino)-2-oxoethylester;
- [1-(Napthalin-2-ylmethyl)piperidin-4-yl]methylcarbamidsäure-2-(methylamino)-2-oxoethylester;
- 2-{1-[(4-Bromphenyl)methyl]piperidin-4-yl}ethylcarbamidsäure-2-(methylamino)-2-oxoethylester;
- 2-(1-{[3-(Trifluormethyl)phenyl]methyl}piperidin-4-yl]ethylcarbamidsäure-2-(methylamino)-2-oxoethylester;
- 2-(1-{[4-(Trifluormethyl)phenyl]methyl}piperidin-4-yl]ethylcarbamidsäure-2-(methylamino)-2-oxoethylester;
- 2-{1-[(2,3-Dichlorphenyl)methyl]piperidin-4-yl}ethylcarbamidsäure-2-(methylamino)-2-oxoethylester;
- 2-{1-[(3,4-Dichlorphenyl)methyl]piperidin-4-yl}ethylcarbamidsäure-2-(methylamino)-2-oxoethylester;
- 2-[1-(Napthalin-2-ylmethyl)piperidin-4-yl]ethylcarbamidsäure-2-(methylamino)-2-oxoethylester;
- 2-[1-(Napthalin-2-ylmethyl)piperidin-4-yl]ethylcarbamidsäure-2-(methylamino)-2-oxoethylester;
- 2-[1-(Pyridin-2-ylmethyl)piperidin-4-yl]ethylcarbamidsäure-2-(methylamino)-2-oxoethylester;
- (1-{2-[4-Fluor-2-(methyloxy)phenyl]ethyl}piperidin-4-yl]methylcarbamidsäure-2-(methylamino)-2-oxoethylester;
- (1-{2-[4-Fluorphenyl)oxy]ethyl}piperidin-4-yl]-carbamidsäure-2-(methylamino)-2-oxoethylester;
- (1-12-[4-Chlorphenyl)oxylethyl}piperidin-4-yl]-carbamidsäure-2-(methylamino)-2-oxoethylester;
- (1-{2-[2,4-Dichlorphenyl)oxy]ethyl}piperidin-4-yl]carbamidsäure-2-(methylamino)-2-oxoethylester;
- (1-{2-[4-Chlorphenyl)oxy]ethyl}piperidin-4-yl]-methylcarbamidsäure-2-(methylamino)-2-oxoethylester;
- (1-{2-[2,4-Dichlorphenyl)oxy]ethyl}piperidin-4-yl]methylcarbamidsäure-2-(methylamino)-2-oxoethylester;
- 2-(1-{2-[4-Fluorphenyl)oxy]ethyl}piperidin-4-yl]ethylcarbamidsäure-2-(methylamino)-2-oxoethylester;
- 2-(1-{2-[4-Chlorphenyl)oxy]ethyl}piperidin-4-yl]ethylcarbamidsäure-2-(methylamino)-2-oxoethylester;
- (1-{2-[4-Fluorphenyl)oxy]ethyl}pyrrolidin-3-yl]-methylcarbamidsäure-2-(methylamino)-2-oxoethylester;
- (1-{2-[4-Chlorphenyl)oxy]ethyl}pyrrolidin-3-yl]-methylcarbamidsäure-2-(methylamino)-2-oxoethylester;
- (1-{2-[3-(Trifluormethyl)phenyl]ethyl}piperidin-4-yl]carbamidsäure-2-(methylamino)-2-oxoethylester;
- {1-[2-(4-Chlorphenyl)ethyl]piperidin-4-yl}carbamidsäure-2-(methylamino)-2-oxoethylester;
- {1-[2-(4-Cyanophenyl)ethyl]piperidin-4-yl}carbamidsäure-2-(methylamino)-2-oxoethylester;
- (1-{2-[(Isochinolin-5-yl)oxy]ethyl}piperidin-4-yl)carbamidsäure-2-(methylamino)-2-oxoethylester;
- [1-(2-Napthalin-1-ylethyl)piperidin-4-yl]-carbamidsäure-2-(methylamino)-2-oxoethylester;
- [1-(2-Napthalin-2-ylethyl)piperidin-4-yl]-carbamidsäure-2-(methylamino)-2-oxoethylester;
- {1-[3-(4-Chlorphenyl)propyl]piperidin-4-yl}carbamidsäure-2-(methylamino)-2-oxoethylester;
- (1-{3-[4-(Methyloxy)phenyl]propyl}piperidin-4-yl)carbamidsäure-2-(methylamino)-2-oxoethylester;
- {1-[2-(3-Chlorphenyl)ethyl]piperidin-4-yl}methylcarbamidsäure-2-(methylamino)-2-oxoethylester;
- (1-{2-[4-(Ethyloxy)phenyl]ethyl}piperidin-4-yl)-methylcarbamidsäure-2-(methylamino)-2-oxoethylester;
- (1-{2-[4-(Dimethylamino)phenyl]ethyl}piperidin-4-yl)methylcarbamidsäure-2-(methylamino)-2-oxoethylester;
- {1-[2-(2,4-Dichlorphenyl)ethyl]piperidin-4-yl}carbamidsäure-2-(methylamino)-2-oxoethylester;
- [1-(2-Napthalin-1-ylethyl)piperidin-4-yl]-methylcarbamidsäure-2-(methylamino)-2-oxoethylester;
- [1-(2-Napthalin-2-ylethyl)piperidin-4-yl]-methylcarbamidsäure-2-(methylamino)-2-oxoethylester;
- (1-{3-[4-(Methyloxy)phenyl]propyl}piperidin-4-yl)methylcarbamidsäure-2-(methylamino)-2-oxoethylester;
- 2-{1-[2-(2-Chlorphenyl)ethyl]piperidin-4-yl}ethylcarbamidsäure-2-(methylamino)-2-oxoethylester;
- 2-{1-[2-(4-Fluorphenyl)ethyl]piperidin-4-yl}ethylcarbamidsäure-2-(methylamino)-2-oxoethylester;
- 2-(1-{2-[4-(Ethyloxy)phenyl]ethyl}piperidin-4-yl]ethylcarbamidsäure-2-(methylamino)-2-oxoethylester;
- 2-{1-[2-(2-Chlor-6-fluorphenyl)ethyl]piperidin-4-yl}ethylcarbamidsäure-2-(methylamino)-2-oxoethylester;
- 2-(1-{3-[4-(Methyloxy)phenyl]propyl}piperidin-4-yl]ethylcarbamidsäure-2-(methylamino)-2-oxoethylester.

9. Verfahren zur Herstellung einer Verbindung der Formel (I) nach einem der Ansprüche 1 bis 8, bei dem man
das Oxazolidindion der allgemeinen Formel (IIa) 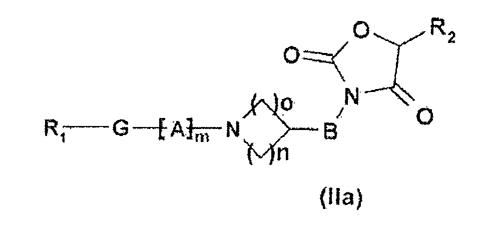 worin A, B, G, R₁, R₂, m, n und o die in der Formel (I) nach Anspruch 1 angegebene Bedeutung besitzen,
durch Aminolyse mit einem Amin der allgemeinen Formel R₃NH₂, worin R₃ die in der Formel (I) nach Anspruch 1 angegebene Bedeutung besitzt, umwandelt.

10. Verfahren zur Herstellung einer Verbindung der Formel (I) nach einem der Ansprüche 1 bis 8, bei dem man das Carbamatamid der allgemeinen Formel (Ia) 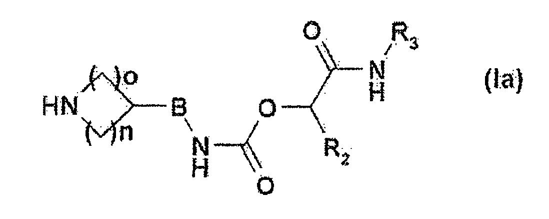 worin B, R₂, R₃, n und o die in der Formel (I) nach Anspruch 1 angegebene Bedeutung besitzen,
durch Umsetzung mit einem Derivat der allgemeinen Formel (III) 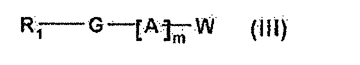 worin W für eine Mesylat- oder Tosylatgruppe oder ein Chlor-, Brom- oder Iodatom steht und m, G, A und R₁ die in der Formel (I) angegebene Bedeutung besitzen, umwandelt.

11. Verbindung der allgemeinen Formel (IIa) 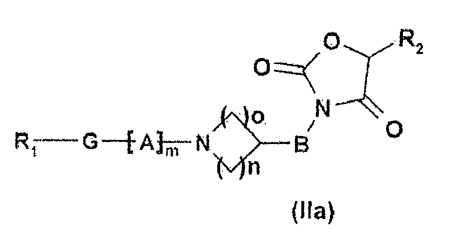 worin A, B, G, R₁, R₂, m, n und o die in der Formel (I) nach Anspruch 1 angegebene Bedeutung besitzen,
wobei 3-[1-(Phenylmethyl)-4-piperidinyl]-2,4-oxazolidindion ausgeschlossen ist.

12. Verbindung der allgemeinen Formel (Ia) 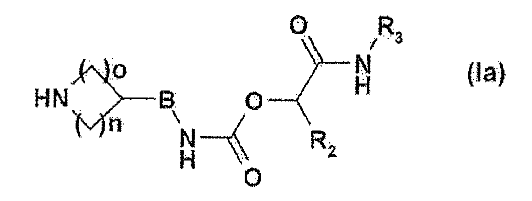 worin B, R₂, R₃, n und o die in der Formel (I) nach Anspruch 1 angegebene Bedeutung besitzen.

13. Pharmazeutische Zusammensetzung, enthaltend mindestens eine Verbindung der Formel (I) nach einem der Ansprüche 1 bis 8 in pharmazeutisch unbedenklicher Basen-, Säureadditionssalz-, Hydrat- oder Solvatform und gegebenenfalls einen oder mehrere pharmazeutisch unbedenkliche Hilfsstoffe.

14. Verbindung der Formel (I) nach einem der Ansprüche 1 bis 8 in pharmazeutisch unbedenklicher Basen-, Säureadditionssalz-, Hydrat- oder Solvatform zur Verwendung als Arzneimittel.

15. Verwendung einer Verbindung der Formel (I) nach einem der Ansprüche 1 bis 8 in pharmazeutisch unbedenklicher Basen-, Salz-, Hydrat- oder Solvatform zur Herstellung eines Arzneimittels zur Prävention oder Behandlung von akuten oder chronischen Schmerzen vom neurogenen Typ, akuten oder chronischen Schmerzen, die mit entzündlichen Erkrankungen assoziiert sind, akuten oder chronischen peripheren Schmerzen, Schwindel, Erbrechen, Übelkeit, Eßstörungen, neurologischen und psychiatrischen Pathologien, akuten und chronischen neurodegenerativen Erkrankungen, Epilepsie, Schlafstörungen, Herz-Kreislauf-Erkrankungen, Nierenischämie, Krebs, Störungen des Immunsystems, allergischen Erkrankungen, parasitären, viralen oder bakteriellen Infektionserkrankungen, entzündlichen Erkrankungen, Osteoporose, Augenleiden, Lungenleiden, Magen-DarmErkrankungen, Harninkontinenz oder Blasenentzündung.
